(19) Europäisches Patentamt
European Patent Office
Office européen des brevets

(11) Veröffentlichungsnummer : **0 399 293 B1**

(12) # EUROPÄISCHE PATENTSCHRIFT

(45) Veröffentlichungstag der Patentschrift :
**01.09.93 Patentblatt 93/35**

(51) Int. Cl.$^5$ : **C07C 205/12**, C07C 201/12

(21) Anmeldenummer : **90108778.3**

(22) Anmeldetag : **10.05.90**

(54) **Verfahren zur Herstellung von 2-Chlor-4-nitro-alkylbenzol.**

(30) Priorität : **23.05.89 DE 3916664**

(43) Veröffentlichungstag der Anmeldung :
**28.11.90 Patentblatt 90/48**

(45) Bekanntmachung des Hinweises auf die Patenterteilung :
**01.09.93 Patentblatt 93/35**

(84) Benannte Vertragsstaaten :
**BE CH DE FR GB IT LI NL**

(56) Entgegenhaltungen :
**CH-A- 648 817**
**DE-A- 3 432 095**
**DE-A- 3 718 060**
**US-A- 3 005 031**
**US-A- 4 069 264**

(73) Patentinhaber : **BAYER AG**
**D-51368 Leverkusen (DE)**

(72) Erfinder : **Mais, Franz-Josef, Dr.**
**Gustav-Poensgen-Strasse 23**
**D-4000 Düsseldorf (DE)**
Erfinder : **Fiege, Helmut, Dr.**
**Walter-Flex-Strasse 23**
**D-5090 Leverkusen 1 (DE)**

**EP 0 399 293 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Monokernchlorierung von 4-Nitro-alkylbenzolen in Gegenwart von Friedel-Crafts-Katalysatoren bei gleichzeitiger Anwesenheit von schwefelhaltigen, benzokondensierten heterocyclischen Co-Katalysatoren in flüssiger Phase.

Die Umsetzung von 4-Nitrotoluol mit gasförmigem Chlor in Gegenwart von Friedel-Crafts-Katalysatoren wie Eisen-III-chlorid (US 3 341 595; J. Chem. Soc 1927, 2905) oder Antimonchloriden (Bull. Soc. Chim Belg. 61 (1952), 317) ist bekannt. Neben dem gewünschten 2-Chlor-4-nitrotoluol werden höherchlorierte, stellungsisomere Dichlor-4-nitrotoluole und 4-Nitro-benzylchlorid sowie die von diesem abgeleiteten Kernchlorderivate als Nebenprodukte beobachtet.

So wird beim Einsatz von Eisen-III-chlorid als Katalysator eine Selektivität von etwa 92 % für das 2-Chlor-4-nitrotoluol beobachtet (siehe Beispiel 2), was den Wunsch nach weiterer Absenkung, insbesondere von 2,6-Dichlor-4-nitrotoluol und 4-Nitro-benzylchlorid aufkommen ließ. Mit Hilfe von Co-Katalysatoren hat man versucht, die Selektivität im gewünschten Sinne weiter zu steigern. Besonders häufig wurde hierbei mit Eisen oder Eisen-III-chlorid und Iod als Co-Katalysator gearbeitet (Naturwiss. 17 (1929), 13; Houben-Weyl, Methoden der Organischen Chemie, Band V/3 (1962), 704; JP-B-75/7589; CS 193 662). Eine Katalysatorkombination Eisen/Iod/$PCl_3$ im Gewichtsverhältnis von 60:1:2 ist aus US 3 005 031 bekannt.

Durch die Mitverwendung von Iod kann zwar die Selektivität zu 2-Chlor-4-nitrotoluol auf über 96 % gesteigert werden (siehe Beispiel 3), jedoch werden hierbei erhebliche Nachteile eingetauscht. Zum einen wird das eingebrachte Iod mit dem reichlich gebildeten Chlorwasserstoff in Form flüchtiger Verbindungen, beispielsweise als Iodwasserstoff, ausgetragen, was die Aufarbeitung und die Reinheit der als Nebenprodukt anfallenden Salzsäure beeinträchtigt. Ferner bleiben Spuren von Iod in gebundener Form, die durch physikalische Trennmethoden nicht entfernbar sind, im Chloriergemisch und im Endprodukt 2-Chlor-4-nitrotoluol. Diese Verunreinigungen stören die Weiterverarbeitung auch des destillierten 2-Chlor-4-nitrotoluols erheblich und bedeuten daher eine beträchtliche Minderung der Produktqualität. Bei der destillativen Aufarbeitung solcher Chloriergemische kann elementares Iod in weiter entfernte Teile der Destillationsapparatur sublimieren; ferner kommt es zur wenig günstigen Bildung von iodhaltigen Aufarbeitungsrückständen.

Ein aus DE-OS 31 28 442 bekanntes Verfahren zur Chlorierung von 4-Nitrotoluol mit Iod als alleinigem Katalysator in einer Menge von beispielsweise 1 Gew.-%, bezogen auf das Einsatzprodukt, zeigt die geschilderten Nachteile der Verwendung von Iod wegen der vergrößerten Menge im besonderen Maße. Zusätzlich entweichen bei diesem zuletztgenannten Verfahren bis zu 75 % des eingeleiteten Chlors ungenutzt aus der Reaktionsmischung; bis zur Erreichung eines industriell erforderlichen Umsatzes von beispielsweise mehr als 90 % wird dadurch ein Überschuß über die benötigte Menge an Chlor von bis zu 300 % erforderlich.

Ein weiteres Verfahren zur Chlorierung von aromatischen Kohlenwasserstoffen, unter denen auch 4-Nitrotoluol aufgeführt wird, wird in CA 1 176 654 beschrieben; hierbei wird in einer Mischung mit 96,5 %iger Schwefelsäure (Gewichtsverhältnis $H_2SO_4$:4-Nitrotoluol = 1:1,35) mit oder ohne Zusatz von geringen Mengen an $Al_2O_3$ gearbeitet. Nähere Angaben zur Zusammensetzung des Chloriergemisches oder zur Ausbeute sind nicht beschrieben. Verfahren mit solchen Mengen an Schwefelsäure sind jedoch technologisch wegen der Korrosion und ökologisch wegen der Entsorgung äußerst ungünstig. Verfahrenstechnisch ist weiterhin eine wäßrige Aufarbeitung zur Entfernung der Schwefelsäure vor einer eventuellen Feindestillation erforderlich.

Es bestand daher der Wunsch, Katalysatorsysteme zur Erhöhung der Selektivität an 2-Chlor-4-nitroalkylbenzolen, d.h. zur Unterdrückung der genannten unerwünschten Nebenprodukte, insbesondere der Höherchlorierung, zu finden, die die oben geschilderten Nachteile nicht aufweisen. Dieses Ziel konnte durch die Kombination von Friedel-Crafts-Katalysatoren mit den weiter unten beschriebenen dibenzokondensierten Schwefelheterocyclen als Co-Katalysatoren in äußerst günstiger Weise erreicht werden.

Die Erfindung betrifft demnach ein Verfahren zur Herstellung von 2-Chlor-4-nitro-alkylbenzol der Formel

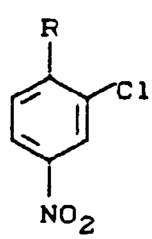

(I),

in der

R für $C_1$-$C_4$-Alkyl steht,

durch Umsetzung des zugrundeliegenden 4-Nitro-alkylbenzols mit elementarem Chlor oder Chlor abgebenden Verbindungen in Gegenwart eines Friedel-Crafts-Katalysators und eines Co-Katalysators in flüssiger Phase, das dadurch gekennzeichnet ist, daß als Co-Katalysator ein dibenzokondensierter Schwefelheterocyclus der Formel

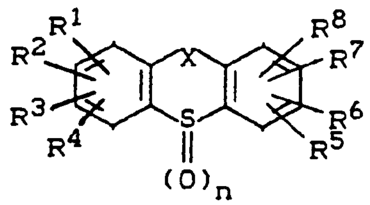

(II)

eingesetzt wird, worin

$R^1$, $R^2$, $R^5$ und $R^6$ unabhängig voneinander Wasserstoff, Halogen, Hydroxy, Cyano, Nitro, Amino, Carboxyl, Halogencarbonyl, die Sulfonsäuregruppe, Halogensulfonyl, $R^9$, $OR^9$, $SR^9$, $COR^9$, $OCOR^9$, $COOR^9$, $NHCOR^9$, $SCOR^9$, $SO_2R^9$ oder $SO_2$-$OR^9$ bedeuten, wobei $R^9$ für geradkettiges oder verzweigtes $C_1$-$C_8$-Alkyl, $C_5$-$C_8$-Cycloalkyl, $C_6$-$C_{12}$-Aryl oder $C_7$-$C_{10}$-Aralkyl steht,

$R^3$, $R^4$, $R^7$ und $R^8$ unabhängig voneinander Wasserstoff, Halogen oder geradkettiges oder verzweigtes $C_1$-$C_8$-Alkyl oder durch die Ethergruppe -O- bzw. durch die Estergruppen -OCO- oder -COO- unterbrochenes $C_1$-$C_8$-Alkyl bedeuten, wobei weiterhin zwei benachbarte Reste von $R^1$ bis $R^4$ bzw. von $R^5$ bis $R^8$ gemeinsam einen anellierten Ring bilden können, der 5 bis 8 Ringglieder aufweisen kann und gesättigt, ungesättigt, aromatisch oder unter Einbeziehung eines oder zweier Atome aus der Gruppe N, O und S heterocyclisch sein kann, und wobei weiterhin zwei benachbarte Reste $R^1$ und $R^2$ bzw. $R^5$ und $R^6$ eine Dicarbonsäureanhydridgruppe darstellen können,

X für -O-, -S-, -SO-, $-SO_2-$ oder eine einfache Bindung steht und

n den Wert Null oder Eins annimmt.

Unter die erfindungsgemäß einzusetzenden dibenzokondensierten Schwefelheterocyclen der Formel (II) fallen die im folgenden lediglich durch ihr durchnumeriertes Grundgerüst angegebenen Verbindungsklassen der Phenoxathiine (III), der Thianthrene (IV), der Thianthren-5-oxide (V), der Thianthren-5,5-dioxide (VI), der Dibenzothiophene (VII) mit dem Index n in der Bedeutung von Null sowie die (nicht numerierten) Verbindungklassen der Phenoxathiin-10-oxide (VIII), der mit (V) identischen Thianthren-10-oxide (IX), der Thianthren-5,10-dioxide (X), der Thianthren-5,5,10-trioxide (XI) und der Dibenzothiophen-5-oxide (XII), wenn der Index n die Bedeutung Eins annimmt:

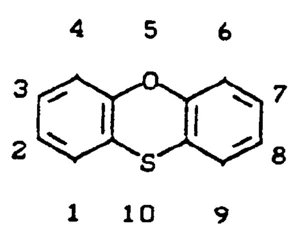

(III)

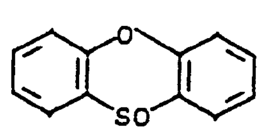

(VIII)

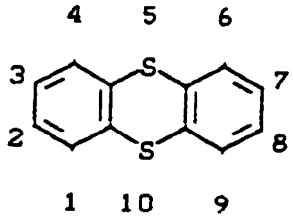

(IV)

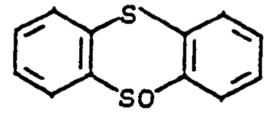

(IX)

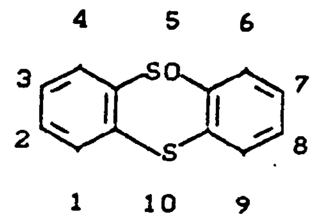 (V) 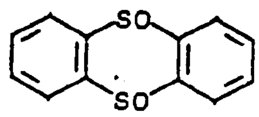 (X)

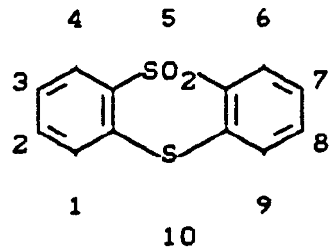 (VI) 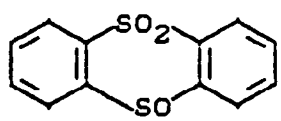 (XI)

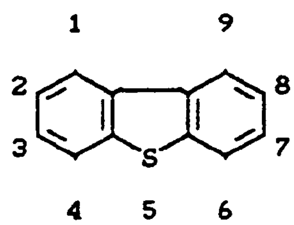 (VII) 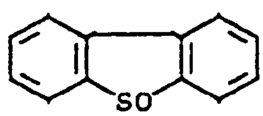 (XII)

Als Halogen sei Fluor, Chlor, Brom oder Iod, bevorzugt Fluor, Chlor oder Brom, besonders bevorzugt Chlor oder Brom genannt. Als Halogencarbonyl sei Fluorcarbonyl, Chlorcarbonyl oder Bromcarbonyl, bevorzugt Chlorcarbonyl, genannt. Als Halogensulfonyl sei Fluorsulfonyl, Chlorsulfonyl oder Bromsulfonyl, bevorzugt Chlorsulfonyl, genannt.

Als geradkettiges oder verzweigtes $C_1$-$C_8$-Alkyl sei Methyl, Ethyl, Propyl, Isopropyl, Butyl, Isobutyl, tert.-Butyl, die isomeren Amyle, Hexyle oder Octyle genannt. Bevorzugtes Alkyl hat 1-4, besonders bevorzugtes Alkyl 1 oder 2 C-Atome.

$C_5$-$C_8$-Cycloalkyl ist beispielsweise Cyclopentyl, Methyl-cyclopentyl, Cyclohexyl, Methyl-cyclohexyl, Cycloheptyl oder Cyclooctyl, in bevorzugter Weise Cyclopentyl oder Cyclohexyl.

$C_6$-$C_{12}$-Aryl ist beispielsweise Phenyl, Naphthyl oder Biphenylyl, bevorzugt Phenyl.

$C_7$-$C_{10}$-Aralkyl ist beispielsweise Benzyl, Phenylethyl, Phenylpropyl oder Phenylbutyl, bevorzugt Benzyl.

Durch die Ethergruppe -O- bzw. durch die Estergruppen -OCO- oder -COO- unterbrochenes $C_1$-$C_8$-Alkyl ist beispielsweise $-CH_2-OCH_3$, $-CH_2-OCO-CH_3$, $-CH_2-CO-OCH_3$ und die entsprechenden Substituenten, in denen $-CH_2-$ bzw. $-CH_3$ durch höhere Alkylen- bzw. Alkylgruppen im genannten Umfang ersetzt sind.

Zwei benachbarte Reste $R^1$ bis $R^4$ bzw. $R^5$ bis $R^8$ auf einem oder auf beiden der kondensierten Benzolkerne können gemeinsam einen an solch einen Benzolkern anellierten Ring bilden, der 5 bis 8 Ringglieder enthalten kann und gesättigt, ungesättigt, aromatisch oder heterocyclisch sein kann, wobei anellierte heterocyclische Ringe 1 oder 2 Atome aus der Gruppe N, O und S enthalten können. Hierdurch kann im Rahmen der Verbindungen der Formel (II) anstelle eines kondensierten Benzolrings beispielsweise ein mit dem aromatischen Teil kondensiertes Indangerüst, Indengerüst, Tetralingerüst, Naphthalingerüst, Chinolingerüst oder anderes zweikerniges Benzoheterocyclusgerüst treten, dessen heterocyclischer Teil auch teilweise oder ganz hydriert sein kann.

Es ist weiterhin möglich, daß die beiden Reste $R^1$ und $R^2$ bzw $R^5$ und $R^6$, wenn sie benachbart sind, gemeinsam eine Dicarbonsäureanhydridgruppe darstellen.

Die genannten Alkyl-, Cycloalkyl-, Aryl- oder Aralkylreste können ihrerseits substituiert sein, beispielsweise durch Methoxy, Ethoxy, Fluor, Chlor, Brom, Trichlormethyl, Trifluormethyl und, besonders in den aromatischen Teilen, durch Nitro, Hydroxy, Amino oder Cyano.

Beispiele für die Vielzahl der erfindungsgemäß einsetzbaren Co-Katalysatoren sind im folgenden aufgezählt:
Thianthren,

2,7-Dichlorthianthren,
2-Chlorthianthren,
2,3,7,8-Tetrachlorthianthren,
1,2,3,4-Tetrachlorthianthren,
Octachlorthianthren,
2,8-Dichlorthianthren,
2,7-Dibromthianthren,
2,3,7-Tribromthianthren,
2,3,7,8-Tetrabromthianthren,
1-Bromthianthren,
2,3-Difluorthianthren,
2,7-Difluorthianthren,
2,8-Difluorthianthren,
2,3,7,8-Tetrafluorthianthren,
1,2,3,7,8,9-Hexafluorthianthren,
Octafluorthianthren,
2,7-Diiodthianthren,
2,7-Dichlor-3,8-dibromthianthren,
2,7-Difluor-3,8-dichlorthianthren,
2-Methyl-thianthren,
2,7-Dimethylthianthren,
2,7-Dimethyl-3,8-dichlorthianthren,
2,8-Dimethyl-3,7-dichlorthianthren,
2,8-Diethylthianthren,
2,7-Dimethyl-3,8-difluor-1,9-dichlorthianthren,
2-Methyl-7-propylthianthren,
2-Hydroxy-1,3,7,8-tetrachlorthianthren,
2-Methoxy-1,7,8-trichlorthianthren,
2,7-Dimethoxythianthren,
2,7-Dimethoxy-3,8-dichlorthianthren,
2,7-Dibrom-3,8-dimethoxythianthren,
2-Methoxy-thianthren,
1-Ethoxythianthren,
1-Ethoxy-2,7-dichlorthianthren,
2-(4-Chlorphenyl)-3,7,8-trichlorthianthren,
2-(4-Methoxymethyl)-3-methyl-7,8-dichlorthianthren,
2-Methoxymethyl-3,7-dichlorthianthren,
2,7-Bis(chlormethyl)-3-chlorthianthren,
2,7-Bis(trifluormethyl)-thianthren,
2,8-Bis(trifluormethyl)-thianthren,
2,7-Bis(trifluormethyl)-3,8-dichlorthianthren,
2-Trifluormethyl-7-cyano-1-methyl-3,8-dichlorthianthren,
2-Trichlormethyl-7-chlor-thianthren,
2-Benzyl-7-cyanothianthren,
2-Benzyl-3,7,8-trichlorthianthren,
2,7-Dibenzylthianthren,
2-Methoxy-3-chlor-7,9-bis(trichlormethyl)-thianthren,
2,7-Bis[(methoxycarbonyl)methyl]thianthren,
2,7-Dichlor-3,8-bis[(methoxycarbonyl)methyl]thianthren,
2,3-Diphenylthianthren,
2-Phenyl-3,8-dichlorthianthren,
2,7-Diphenylthianthren,
2,8-Diphenylthianthren,
2-Phenyl-7-methyl-3,8-dichlorthianthren,
2-(2-Furyl)-thianthren,
2-(2-Thienyl)-3,7,8-trichlorthianthren,
1-Phenoxy-thianthren,
2-Phenoxy-3,7,8-trichlorthianthren,

2,7-Diphenoxythianthren,
2-Cyano-thianthren,
2,7-Dicyanothianthren,
2,8-Dicyanothianthren,
2,3,7,8-Tetracyanothianthren,
2,7-Dicyano-3,8-dichlorthianthren,
2,7-Dicyano-3,8-dichlor-1-methylthianthren,
2-Cyano-3,8-dibrom-thianthren,
2-Nitrothianthren,
2,7-Dinitrothianthren,
2,8-Dinitrothianthren,
1,4-Dinitrothianthren,
1,9-Dimethyl-4,6-dinitrothianthren,
1,6-Dimethyl-4,9-dinitrothianthren,
1,9-Dimethyl-4,6-dinitro-2-chlorthianthren,
1,9-Dimethyl-4,6-dinitro-2,8-dichlorthianthren,
1,9-Dimethyl-4,6-dinitro-2,3,7,8-tetrachlorthianthren,
1,6-Dimethyl-4,9-dinitro-2-chlorthianthren,
1,6-Dimethyl-4,9-dinitro-2,7-dichlorthianthren,
1,6-Dimethyl-4,9-dinitro-2,3,7,8-tetrachlorthianthren,
1,9-Dimethyl-4,6-dinitro-2,8-dibromthianthren,
1,6-Dimethyl-4,9-dinitro-2,7-dibromthianthren,
1,9-Diethyl-4,6-dinitrothianthren,
1,6-Diethyl-4,9-dinitrothianthren,
2,4,6,8-Tetronitrothianthren,
2-Acetylthianthren,
2-Trichloracetyl-thianthren,
2-Trifluoracetyl-thianthren,
2,7-Diacetylthianthren,
2,8-Diacetylthianthren,
2,7-Diacetyl-3,8-dichlorthianthren,
2,7-Diacetyl-1,3,6,8-tetrachlorthianthren,
2-Acetyl-7-trifluoracetyl-thianthren,
2-Propionyl-3,7-dichlorthianthren,
1,4-Diacetyl-2,3,7,8-tetrachlorthianthren,
2,7-Dibenzoylthianthren,
2,8-Dibenzoylthianthren,
2,7-Dibenzoyl-3,8-dichlorthianthren,
2,7-Diacetyl-3,8-dicyanothianthren,
1,4-Dichlor-2-acetylthianthren,
2,7-Dinitro-3-trifluoracetyl-thianthren,
2,7-Dibrom-2,8-bis(trifluoracetyl)-thianthren,
1-Chlor-2-benzoyl-3,7-dibromthianthren,
2,7-Bis(acetoxy)-thianthren,
2,7-Dichlor-3,8-bis(acetyloxy)-thianthren,
1,9-Bis(trifluoracetyloxy)-2,8-dichlorthianthren,
2-Benzoyloxythianthren,
2-Benzoyl-7-acetyl-3,8-dichlorthianthren,
2-Acetyloxy-1,3,6,7,8-pentachlorthianthren,
2,7-Bis(acetylamino)-thianthren,
2,7-Acetoy-3,8-dimethyl-1,4,6,9-tetrachlorthianthren,
2,8-Bis(acetylamino)-thianthren
2,7-Bis(acetylamino)-3,8-dichlorthianthren,
2,8-Bis(acetylamino)-1,3,7,9-tetrachlorthianthren,
2-Acetylamino-3,8-dicyano-6,7,9-trichlorthianthren,
2-Carboxylthianthren,
2,7-Dicarboxyl-3,8-dichlorthianthren,
2,8-Dicarboxyl-3,7-dichlorthianthren,

2,7-Bis(methoxycarbonyl)-thianthren,
2,8-Bis(methoxycarbonyl)-thianthren,
1-Methoxycarbonylthianthren,
2-Methoxycarbonylthianthren,
2,7-Bis(benzoylamino)-thianthren,
2,8-Bis(benzoylamino)-thianthren,
2,7-Bis(methoxycarbonyl)-3,8-dichlorthianthren,
2,8-Bis(methoxycarbonyl)-3,7-dichlorthianthren,
2,7-Bis(chlorcarbonyl)-thianthren,
2,7-Bis(fluorcarbonyl)-1,3,4,6,8,9-hexachlorthianthren,
2,8-Bis(bromcarbonyl)-thianthren,
2,8-Bis(chlorcarbonyl)-3,7-dichlorthianthren,
2,7-Bis(chlorcarbonyl )-3,8-dimethyl-1,4,6,9-tetrachlorthianthren,
2,7-Bis(methylthio)-thianthren,
2,7-Bis(phenylthio)-thianthren,
2,7-Bis(methylthio)-1,3,6,8-tetrachlorthianthren,
2,7-Bis(acetylthio)-thianthren,
2,7-Bis(acetylthio)-3,8-bis(methoxycarbonyl )-1,6-dichlorthianthren,
2-Benzoylthio-3,7,8-trichlorthianthren,
2,7-Disulfothianthren,
2,7-Dichlor-3-sulfothianthren,
2,8-Disulfo-1,4,6,9-tetrachlorthianthren,
2,7-Bis(chlorsulfonyl)-thianthren,
2,8-Bis(chlorsulfonyl)-thianthren,
2,7-Bis(fluorsulfonyl)-thianthren,
2,7-Bis(chlorsulfonyl)-3,8-dichlorthianthren,
2,8-Bis(chlorsulfonyl)-3,7-dichlorthianthren,
2,7-Bis(chlorsulfonyl)-1,3,4,6,8,9-hexachlorthianthren,
2-Phenylsulfonyl-thianthren,
2-Tosylthianthren,
2,7-Ditosyl-3,8-dichlorthianthren,
2,7-Dimesyl-3,8-dichlorthianthren,
2,7-Bis(methoxysulfonyl)-3,8-dichlorthianthren,
2,7-Bis(phenoxysulfonyl)-1,3,6,9-tetrachlorthianthren,
1,4,6,9-Tetrachlorthianthren-2,3,7,8-tetracarbonsäuredianhydrid,
1,4,6,9-Tetrabromthianthren-2,3,7,9-tetracarbonsäuredianhydrid,
Cyclopentano[b]-thianthren der Formel

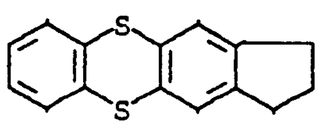 ,

Cyclohexano[b]-thianthren der Formel

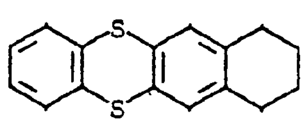 ,

(Diethyl-cyclopentano[b])-thianthren der Formel

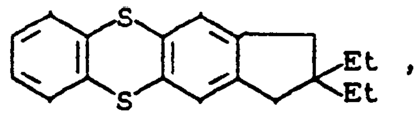

(Diethyl-dioxo-cyclopentano[b]-thianthren der Formel

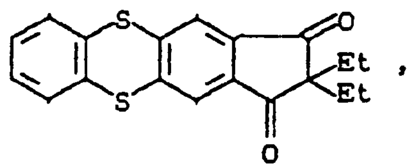

Benzo[b]-thianthren,
7,8-Dichlor-benzo[b]-thianthren,
Thianthren-5-oxid,
Thianthren-5,5-dioxid,
2,3,7,8-Tetrachlorthianthrens-5-oxid,
2,7-Dichlorthianthren-5-oxid,
2,8-Diamino-1,3,7,9-tetrachlorthianthren,
Phenoxathiin,
2,8-Dichlorphenoxathiin,
2,3,7,8-Tetrachlorphenoxathiin,
1,2,3,7,8,9-Hexachlorphenoxathiin,
1,2,3,6,7,8-Hexachlorphenoxathiin,
2,3,4,6,7,7-Hexachlorphenoxathiin,
Octachlorphenoxathiin,
Octafluorphenoxathiin,
2,8-Difluorphenoxathiin,
2,3,7,8-Tetrabromphenoxathiin,
2,8-Difluor-3,7-dichlorphenoxathiin,
2,8-Dimethyl-1,3,7,9-tetrachlorphenoxathiin,
3,9-Dimethyl-2,6,8-trichlorphenoxathiin,
3,6,9-Trimethyl-8-chlor-phenoxathiin,
3,6,9-Trimethyl-1,2,4,7,8-pentachlor-phenoxathiin,
2-Methyl-1,3,7,8-tetrachlorphenoxathiin,
2,8-Diethyl-1,3,7,9-tetrachlorphenoxathiin,
2-Methoxy-1,3,7,8-tetrachlorphenoxathiin,
2,8-Dimethyl-1,3,7,9-tetrabromphenoxathiin,
2,8-Dimethyl-1,3,4,6,7,9-hexachlorphenoxathiin,
3,7-Dimethyl-2,4,6,8-tetrachlorphenoxathiin,
1,7-Dimethyl-2,4,8,9-tetrachlorphenoxathiin,
3,7-Dimethyl-1,2,8,9-tetrachlorphenoxathiin,
4,6-Dimethyl-2,3,7,8-tetrachlorphenoxathiin,
4,6-Dimethyl-1,3,7,9-tetrachlorphenoxathiin,
2,8-Diphenyl-1,3,7,9-tetrachlorphenoxathiin,
2,8-Trifluormethyl-3,7-dichlorphenoxathiin,
2,8-Trifluormethyl-1,3,7,9-tetrabromphenoxathiin,
2-Benzyl-3,7,8-trichlorphenoxathiin,
2-Methoxymethyl-3,7,9-trichlorphenoxathiin,
2,8-Bis[(methoxycarbonyl)methyl]-1,3,7,9-tetrachlorphenoxathiin,
2-Phenoxy-1,3,7,8-tetrachlorphenoxathiin,
2,8-Dicyano-3,7-dichlorphenoxathiin,
1,4-Dicyano-2,3,7,8-tetrachlorphenoxathiin,
2,3,7,8-Tetracyanophenoxathiin,

2-Nitro-1,4,7,8-tetrachlorphenoxathiin,
2-Nitrophenoxathiin,
2,4-Dinitro-7,8-dichlorphenoxathiin,
2,8-Diacetyl-1,3,7,9-tetrachlorphenoxathiin,
2,8-Bis(trifluoracetyl)-3,7-dichlorphenoxathiin,
2,8-Dibenzoyl-1,3,7,9-tetrachlorphenoxathiin,
2,8-Dinitrophenoxathiin,
2,8-Dinitro-3,7-dichlorphenoxathiin,
2,8-Dinitro-3,7-dimethyl-1,9-dichlorophenoxathiin,
2-Acetyl-1,3,7,8-tetrachlorphenoxathiin,
2,8-Bis(acetylamino)-phenoxathiin,
2,8-Bis(trifluoracetylamino)-1,3,7,9-tetrachlorphenoxathiin,
2,8-Bis(methoxycarbonyl)-1,3,7,9-tetrachlorphenoxathiin,
2-Methoxycarbonyl-3,7-dichlorphenoxathiin,
2,8-Bis(methoxycarbonyl)-1,3,6,9-tetrachlorphenoxathiin,
2,8-Bis(chlorcarbonyl)-3,7-dichlorphenoxathiin,
2,8-Bis(fluorcarbonyl)-3,7-dichlorphenoxathiin,
2,8-Bis(chlorcarbonyl)-1,3,4,6,7,9-hexachlorphenoxathiin,
2,8-Bis(methylthio)-1,3,7,9-tetrachlorphenoxathiin,
2,3-Bis(acetylthio)-1,4,7,8-tetrachlorphenoxathiin,
2,8-Bis(chlorsulfonyl)-3,7-dichlorphenoxathiin,
2,8-Bis(chlorsulfonyl)phenoxathiin,
2,8-Bis(chlorsulfonyl)-1,3,7,9-tetrachlorphenoxathiin,
2,8-Bis(fluorsulfonyl)-3,7-dichlorphenoxathiin,
2,8-Bis(chlorsulfonyl)-1,3,4,6,7,9-hexachlorphenoxathiin,
2,8-Bismesyl-1,3,7-trichlorphenoxathiin,
2,8-Bistosyl-1,3,7,9-tetrachlorphenoxathiin,
Benzo[b]-8-nitro-phenoxathiin der Formel

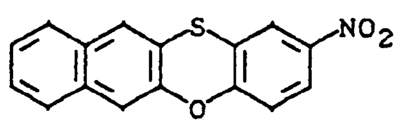

Benzo[b]-1,4,6,9-tetrachlor-8-nitrophenoxathiin der Formel

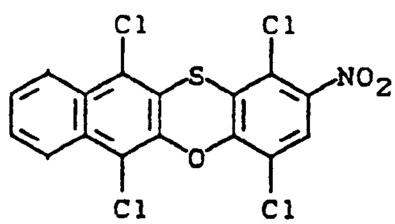

Dibenzo[a,h]-3,8-dichlor-4,9-dibromphenoxathiin der Formel

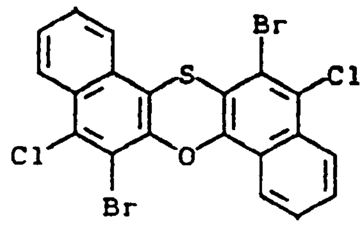

Dibenzothiophen,
2,8-Dichlor-dibenzothiophen,
2,3,7,8-Tetrachlor-dibenzothiophen,
Octachlor-dibenzothiophen,
2-Brom-dibenzothiophen,
2-Nitro-dibenzothiophen,
2-Nitro-7,8-dichlor-dibenzothiophen,
2-Methoxy-3-nitro-dibenzothiophen,
2,8-Difluor-dibenzothiophen,
2,8-Difluor-3,7-dimethyl-dibenzothiophen,
2-Acetylamino-dibenzothiophen,
2-Acetylamino-3-nitro-dibenzothiophen,
2,3-Dibrom-dibenzothiophen,
2,8-Dibrom-dibenzothiophen,
2-Phenylsulfonyl-dibenzothiophen,
2-Phenylsulfonyl-3,7,8-trichlor-dibenzothiophen,
2,8-Bis(chlorsulfonyl)-dibenzothiophen,
2-Nitro-1,3,4,6,7,8,9-heptachlor-dibenzothiophen,
2-Acetylamino-1,3,7,8-tetrachlor-dibenzothiophen,
2-Carboxyl-1,3,7,8-tetrachlor-dibenzothiophen,
2-Carboxyl-dibenzothiophen,
2-Benzoyl-dibenzothiophen,
2-(2,4-Dichlorbenzoyl)-dibenzothiophen,
3,7-Diamino-2,4,6,8-tetrachlor-dibenzothiophen,
Octafluor-dibenzothiophen.

Die Verbindungen der Formel (II) sind dem Fachmann grundsätzlich bekannt und können nach bekannten Methoden hergestellt werden. So kann man Phenoxathiine durch Umsetzung von Diarylethern mit Schwefel bzw. mit Dischwefeldichlorid in Gegenwart von Aluminiumchlorid erhalten (Org. Synth., Coll. Vol. 2, S. 485; J. Am. Chem. Soc. 59 (1937), 2578; EP-173 222; JP-A-56/110 630; J. Am. Chem. Soc. 58 (1936), 717; DE-AS 12 22 508; EP-63 384); eine weitere Herstellungsmethode besteht im Ringschluß von 2-Sulfino-diarylethern (J. Chem. Soc. 123 (1923), 2876).

Thianthrene können beispielsweise durch Umsetzung von aromatischen Verbindungen mit Schwefel oder Dischwefeldichlorid in Gegenwart von Aluminiumchlorid oder durch Kondensation von Arylmercaptanen in konzentrierter Schwefelsäure oder durch Ringschluß von 2-Sulfino-diarylthioethern hergestellt werden (Ann. 381 (1911), 312; J. Chem. Soc. 119 (1921), 1959; DE-OS 27 39 433; Ann. 407 (1915), 194; J. Chem. Soc. 123 (1923), 156).

Dibenzothiophene können beispielsweise durch Umsetzung von Biphenylen mit Schwefel oder Dischwefeldichlorid und Aluminiumchlorid oder aus 2,2'-Dihydroxybiphenylen und Phosphorpentasulfid erhalten werden (DE-PS 579 917, J. Org. Chem. 3 (1938), 108; DE-PS 330 833).

Solche durch Ringaufbaureaktionen erhaltenen Phenoxathiine, Dibenzothiophene oder Thianthrene können nach den bekannten Methoden für die Substitution an aromatischen Kernen noch weiter beispielsweise durch Chlorierung, Sulfochlorierung, Bromierung, Acylierung und andere Umsetzungen in weitere Derivate umgewandelt werden (J. Am. Chem. Soc. 58 (1936), 717; J. Am. Chem. Soc. 75 (1953), 3384; J. Chem. Soc. 1956, 2408; J. Am. Chem. Soc. 77 (1955), 5944; EP-173 222, EP-63 384; DE-OS 27 39 433, DE-AS 12 22 508; Adv. Heterocycl. Chem. 16 (1974), 181).

Bei den genannten Herstellungsweisen erhält man vielfach Gemische stellungsisomerer Derivate. So kann beispielsweise bei der Thianthren-Synthese aus Chlorbenzol, Schwefel und $AlCl_3$ das 2,7-Dichlor-thianthren im Gemisch mit dem 2,8-Dichlor-thianthren gemäß folgender Gleichung entstehen:

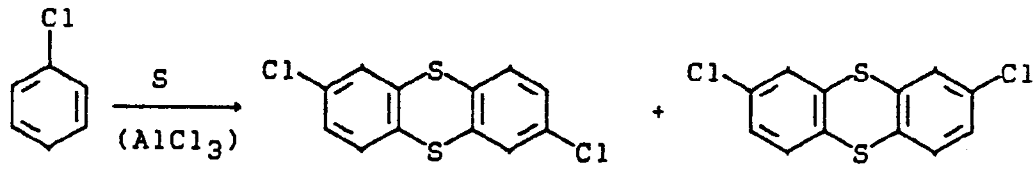


Aus 3-Methyl-diphenylether, $S_2Cl_2$ und $AlCl_3$ entsteht in ähnlicher Weise ein Gemisch aus 1-Methyl- und 3-Methylphenoxathiin gemäß der folgenden Gleichung:

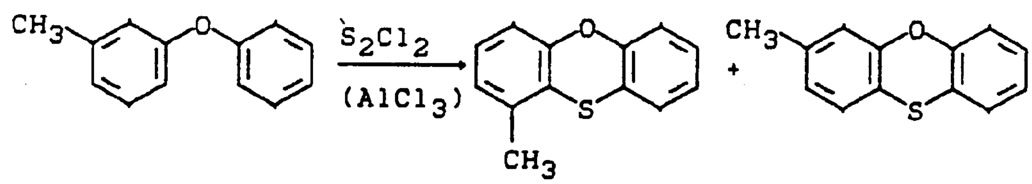


Aus anderen Ausgangsstoffen kann nur ein stellungsisomeres Produkt entstehen:

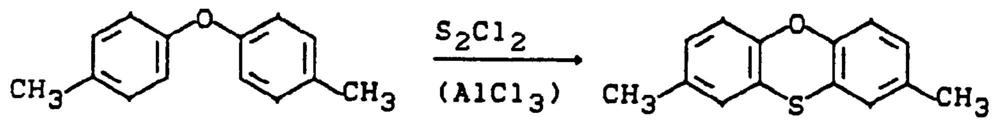


Auch bei der weiteren Derivatisierung der durch Ringaufbau erhaltenen, erfindungsgemäß einsetzbaren Heterocyclen erhält man vielfach Gemische von stellungsisomeren Produkten. So erhält man durch Chlorierung von 2,8-Dimethyl-phenoxathiin auf einen mittleren Chlorierungsgrad von 4 ein Gemisch, das hauptsächlich in beiden Benzolkernen zweimal chloriert ist, allerdings in den denkbaren verschiedenen Stellungsisomeren; daneben treten auch asymmetrisch vierfach chlorierte Produkte auf (EP-63 384). Auch Gemische von dreifach und fünffach chlorierten Molekülen können einen durchschnittlichen Chlorierungsgrad 4 ergeben.

Selbstverständlich sind solche Gemische beim erfindungsgemäßen Einsatz den reinen Verbindungen absolut gleichwertig. Aus ökonomischen Überlegungen wird man solche Gemische sogar bevorzugt einsetzen, ohne sie in die Einzelverbindungen aufzutrennen. Desweiteren sind erfindungsgemäß einsetzbare Co-Katalysatoren auch solche, die aus Verbindungen, die unter die Formel (II) fallen, erst im Chlorierungsgemisch durch Aufchlorieren entstehen. Hierzu gehören neben den beschriebenen kernchlorierten Verbindungen auch solche, die Schwefel-Chlor-Bindungen enthalten und beispielsweise als Thianthren-dithioniumchloride formuliert werden.

Umgekehrt können auch beispielsweise solche Thianthrendithioniumchloride oder -bromide eingesetzt werden, die im Reaktionsgemisch im Sinne eines Gleichgewichts teilweise ihr Chlor (Brom) verlieren und sodann teilweise als Verbindungen der Formel (II) vorliegen.

Zu den erfindungsgemäß einsetzbaren Co-Katalysatoren gehören weiterhin auch offenkettige Vorstufen, die unter den erfindungsgemäßen Bedingungen einen Ringschluß eingehen und damit in erfindungsgemäße Co-Katalysatoren übergehen.

In bevorzugter Weise werden erfindungsgemäß Co-Katalysatoren der Formel

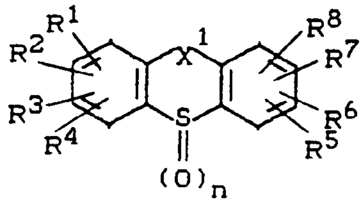

(XIII)

eingesetzt, worin

$X^1$ für -O-, -S- oder eine Einfachbindung steht und

$R^1$ bis $R^8$ und n die oben gegebene Bedeutung haben.

Weitere bevorzugte, erfindungsgemäß einsetzbare Co-Katalysatoren sind solche der Formel

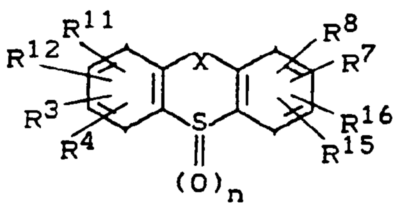

(XIV),

worin

$R^{11}$, $R^{12}$, $R^{15}$ und $R^{16}$ unabhängig voneinander Wasserstoff, Halogen, Cyano, Nitro, Halogencarbonyl, Halogensulfonyl, $R^{19}$, $OR^{19}$, $COOR^{19}$ oder $COR^{19}$ bedeuten, wobei $R^{19}$ für geradkettiges oder verzweigtes $C_1$-$C_8$-Alkyl, Phenyl oder Benzyl steht, wobei weiterhin zwei benachbarte Reste $R^{11}$ und $R^{12}$ bzw. $R^{15}$ und $R^{16}$ eine Dicarbonsäureanhydridgruppe darstellen können, und

$R^3$, $R^4$, $R^7$, $R^8$, X und n die oben angegebene Bedeutung haben.

Besonders bevorzugte, erfindungsgemäß einsetzbare Co-Katalysatoren sind solche der Formel

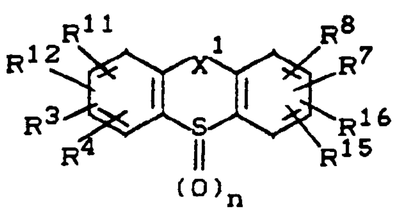

(XV),

worin

$x^1$ für -O-, -S- oder eine Einfachbindung steht und

$R^{11}$, $R^{12}$, $R^3$, $R^4$, $R^{15}$, $R^{16}$, $R^7$, $R^8$ und n die oben gegebene Bedeutung haben.

Weitere besonders bevorzugte erfindungsgemäß einsetzbare Co-Katalysatoren sind solche der Formel

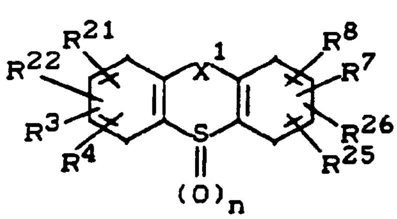

(XVI),

worin

$R^{21}$ und $R^{25}$ unabhängig voneinander Wasserstoff, Fluor, Chlor, Brom, Cyano, Nitro, Fluor-

| | |
|---|---|
| | carbonyl, Chlorcarbonyl, Chlorsulfonyl, $R^{29}$ oder $OR^{29}$ bedeuten, wobei $R^{29}$ für geradkettiges oder verzweigtes $C_1$-$C_4$-Alkyl, Phenyl oder Benzyl steht, |
| $R^{22}$ und $R^{26}$ | unabhängig voneinander Wasserstoff, Fluor, Chlor, Brom oder geradkettiges oder verzweigtes $C_1$-$C_4$-Alkyl bedeuten, wobei weiterhin benachbarte Reste $R^{21}$ und $R^{22}$ gemeinsam bzw. $R^{25}$ und $R^{26}$ gemeinsam eine Dicarbonsäureanhydridgruppe darstellen können, und |
| $R^3$, $R^4$, $R^7$, $R^8$, $X^1$ und n | die oben gegebene Bedeutung haben. |

Ganz besonders bevorzugte, erfindungsgemäß einsetzbare Co-Katalysatoren sind solche der Formel

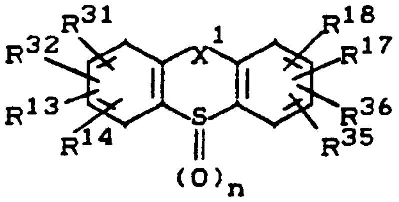

(XVII),

worin

| | |
|---|---|
| $R^{31}$ und $R^{35}$ | unabhängig voneinander Wasserstoff, Fluor, Chlor, Brom, Nitro, Chlorcarbonyl, Methyl, Ethyl, Methoxy, Ethoxy oder Acetyl bedeuten, |
| $R^{32}$ und $R^{36}$ | unabhängig voneinander Wasserstoff, Chlor, Brom, Methyl oder Ethyl bedeuten, wobei weiterhin benachbarte Reste $R^{31}$ und $R^{32}$ gemeinsam bzw. $R^{35}$ und $R^{36}$ gemeinsam eine Dicarbonsäureanhydridgruppe darstellen können, |
| $R^{13}$, $R^{14}$, $R^{17}$ und $R^{18}$ | unabhängig voneinander Wasserstoff, Chlor, Brom, Methyl, $-CH_2-OCH_3$ oder $-CH_2COOCH_3$ bedeuten, |
| $X^1$ | für -O-, -S- oder eine Einfachbindung steht und |
| n | den Wert Null oder Eins annimmt. |

Weitere bevorzugte Co-Katalysatoren sind solche der Formeln (XIII), (XV), (XVI) und (XVII), in denen an die Stelle von $X^1$ das Ringglied $X^2$ mit der Bedeutung -O-oder -S- tritt.

Von allen genannten Verbindungsklassen sind diejenigen bevorzugt, in denen der Index n den Wert Null annimmt.

Beispiele für die erfindungsgemäß in 2-Position einfach zu chlorierenden Verbindungen der Formel (I) sind: 4-Nitro-toluol, 4-Nitro-ethylbenzol, 4-Nitro-propylbenzol und 4-Nitro-butylbenzol; besonders wichtig ist das Verfahren für die Chlorierung von 4-Nitro-toluol.

Das erfindungsgemäße Verfahren wird in flüssiger Phase durchgeführt, wobei das 4-Nitro-alkylbenzol (I) in flüssiger (geschmolzener) Form oder gegebenenfalls in Verdünnung mit einem inerten Lösungsmittel eingesetzt wird. Geeignete Lösungsmittel sind solche, die durch Chlor oder durch das Katalysatorsystem unter den Bedingungen des erfindungsgemäßen Verfahrens nicht angegriffen werden; solche Lösungsmittel sind dem Fachmann grundsätzlich bekannt und umfassen beispielsweise Methylenchlorid, Chloroform, Tetrachlorkohlenstoff und Essigsäure. In bevorzugter Weise wird ohne Lösungsmittel gearbeitet.

Als Chlorierungsmittel für das erfindungsgemäße Verfahren wird vorzugsweise Chlor eingesetzt, das flüssig oder gasförmig in das Reaktionsgemisch eingeleitet werden kann; bevorzugt wird gasförmiges Chlor eingeleitet. Es können jedoch auch andere Chlorierungsmittel verwendet werden, die unter den Reaktionsbedingungen Chlor abgeben, wie beispielsweise Sulfurylchlorid.

Das erfindungsgemäße Verfahren kann grundsätzlich bei einer Temperatur vom Erstarrungspunkt bis zum Siedepunkt des Reaktionsgemisches durchgeführt werden. Im allgemeinen liegt die Reaktionstemperatur bei 50-150°C, bevorzugt bei 70-120°C, besonders bevorzugt bei 80-100°C. Der Reaktionsdruck kann normal, vermindert oder erhöht sein und ist grundsätzlich unkritisch. Die Durchführung bei Normaldruck ist wegen der Vereinfachung der Reaktionsapparatur bevorzugt. Eine Erhöhung des Druckes kann dann angezeigt sein, wenn ein niedrigsiedendes Reaktionsgemisch (niedrigsiedendes Lösungsmittel) in der flüssigen Phase gehalten werden soll. In diesem Fall wird in bevorzugter Weise unter dem sich automatisch einstellenden Eigendruck eines solchen Reaktionsgemisches gearbeitet.

Das erfindungsgemäße Verfahren ist auf die Herstellung eines 2-Chlor-4-nitro-alkylbenzols, also auf die Herstellung einer monochlorierten Verbindung, gerichtet. Für eine solche Monochlorierung werden 80-110 Mol-%, bevorzugt 80-100 Mol-% Chlor in elementarer Form oder in Form einer Chlor abgebenden Substanz pro Mol 4-Nitroalkylbenzol eingesetzt.

Ein geringer Wassergehalt der Reaktionsmischung ist im allgemeinen unkritisch. In bevorzugter Weise brauchen daher alle Einsatzstoffe nicht speziell getrocknet zu werden. Selbstverständlich ist eine solche vollständige Trocknung einzelner oder aller Einsatzstoffe möglich. Unter einem geringen Wassergehalt wird ein solcher verstanden, der nicht über den Sättigungsgrenzen der jeweiligen Einsatzstoffe liegt. Fernerhin darf ein solch geringer Wassergehalt nicht so groß sein, daß der eingesetzte Friedel-Crafts-Katalysator durch Hydrolyse vollständig aufgebraucht wird. Beispielhaft zu nennende geringe Wassergehalte im Reaktionsgemisch sind solche bis 250 ppm, bevorzugt bis 150 ppm.

Friedel-Crafts-Katalysatoren für das erfindungsgemäße Verfahren sind alle dem Fachmann bekannten Katalysatoren, wie Antimonchloride, Aluminiumchlorid oder Eisenchloride. Es können jedoch auch Elemente oder Elementverbindungen eingesetzt werden, die während der Chlorierung einen Friedel-Crafts-Katalysator (Lewis-Säure) bilden. Hierzu gehören beispielsweise die Elemente Eisen, Antimon, Aluminium oder Zink, sowie die Oxide, Sulfide oder Carbonyle dieser Elemente, fernerhin Salze schwacher Säuren, wie die Carbonate; beispielhaft seien Antimonoxide, Eisenoxide, Eisensulfide, Eisencarbonyle, Eisencarbonate oder ähnliche genannt. Anstelle der erwähnten Chloride können auch die Bromide, gegebenenfalls auch die Fluoride der genannten Elemente eingesetzt werden. Bevorzugte Friedel-Crafts-Katalysatoren sind Antimonchloride und Eisenchloride, besonders bevorzugt ist Eisen-III-chlorid.

Die Einsatzmengen des Friedel-Crafts-Katalysators oder eines Gemisches mehrerer von ihnen können in weiten Grenzen variiert werden. So ist bereits bei einem Zusatz von 0,005 Gew.-% eine Katalysatorwirkung erkennbar. Andererseits können auch 10 Gew.-% oder mehr des Friedel-Crafts-Katalysators zugesetzt werden, jedoch bieten solche hohen Mengen im allgemeinen keinen Vorteil, sind aber kostenintensiv und bringen gegebenenfalls bei der Aufarbeitung Schwierigkeiten. Üblicherweise wird der Friedel-Crafts-Katalysator in einer Menge von 0,01-3 Gew.-%, bevorzugt von 0,05-1,5 Gew.-%, besonders bevorzugt von 0,1-0,75 Gew.-% eingesetzt. Alle Mengenangaben sind auf die Menge des eingesetzten 4-Nitro-alkylbenzols bezogen.

Die erfindungsgemäßen Co-Katalysatoren oder Gemische mehrerer von ihnen oder genannte Vorstufen können in einer in weiten Grenzen variierbaren Menge eingesetzt werden. Bei Mengen unter 0,01 Gew.-% läßt jedoch die Co-katalytische Wirkung nach. Mengen über 10 Gew.-% bieten keinen weiteren Vorteil, sind jedoch kostenintensiv und können die Aufarbeitung erschweren. Im allgemeinen werden die erfindungsgemäßen Co-Katalysatoren daher in Mengen von 0,01-5 Gew.-%, bevorzugt 0,05-2,5 Gew.-%, besonders bevorzugt 0,1-1 Gew.-%, jeweils bezogen auf das eingesetzte 4-Nitro-alkylbenzol, eingesetzt.

Das Molverhältnis der eingesetzten Friedel-Crafts-Katalysatoren zu den Co-Katalysatoren kann im erfindungsgemäßen Verfahren in weiten Grenzen variiert werden. Im allgemeinen ist es vorteilhaft, den Co-Katalysator nicht in zu großem Überschuß und nicht in zu großem Unterschuß gegenüber dem Friedel-Crafts-Katalysator einzusetzen. Im allgemeinen wird daher ein molares Verhältnis von Friedel-Crafts-Katalysator zu Co-Katalysator von 50: 1-1:10, bevorzugt 10:1-1:2, besonders bevorzugt 3:1-1:1,1 gewählt.

Für die praktische Durchführung des erfindungsgemäßen Verfahrens ist die Reihenfolge der Zugabe der einzelnen Komponenten des Reaktionsgemisches beliebig. Hierbei läßt sich das Verfahren sowohl kontinuierlich als auch diskontinuierlich durchführen. Eine beispielhafte Ausführungsform ist die folgende:

Das gewünschte 4-Nitro-alkylbenzol, beispielsweise 4-Nitro-toluol, wird vorgelegt und beispielsweise auf 90°C erwärmt. Dann gibt man in beliebiger Reihenfolge die gewünschten Mengen an Friedel-Crafts-Katalysator(en) und Co-Katalysator(en) zu und leitet unter weitgehender Konstanthaltung der Temperatur Chlor gasförmig in der vorgewählten Menge ein. Anschließend wird das Gemisch in üblicher Weise, etwa durch Destillation aufgearbeitet.

Eine weitere beispielhafte Ausführungsform ist die folgende:

Man stellt eine Lösung von Katalysator und Co-Katalysator in dem gewünschten 4-Nitro-alkylbenzol her und bringt diese auf die gewünschte Reaktionstemperatur. Dann wird ein Chlorierungsmittel in der vorgesehenen Menge zugesetzt. Auch hier kann die Aufarbeitung durch Destillation erfolgen.

Das erfindungsgemäße Verfahren ist durch eine ausgeprägte Stufenselektivität in Bezug auf die Monochlorierung ausgezeichnet. Desweiteren ist der erfindungsgemäße Einsatz der oben beschriebenen Co-Katalysatoren durch eine aktivierende Wirkung auf den Chlorierungsverlauf ausgezeichnet, so daß die Verluste durch nicht umgesetztes und das Reaktionsgemisch wieder verlassendes Chlor stark herabgesetzt werden. Diese Aktivierung schien mit einer Selektivität bezüglich einer Monochlorierung nicht vereinbar zu sein. Noch weiterhin ist es überraschend, daß diese guten Ergebnisse bei einer technisch sehr vorteilhaften Temperatur im obengenannten Bereich erzielt werden.

Weiterhin vorteilhaft ist ferner die Tatsache, daß die erfindungsgemäßen Co-Katalysatoren insbesondere mit dem technisch außerordentlich günstigen und wünschenswerten Friedel-Crafts-Katalysator Eisen-III-chlorid die genannten guten Ergebnisse liefern.

Bei den folgenden Ausführungsbeispielen wird die Selektivität für die Bildung des 2-Chlor-4-nitro-alkylbenzols wie folgt definiert:

$$\text{Selektivität} = \frac{[\text{Gehalt an } 2-\text{Cl}-4-\text{NO}_2-\text{alkylbenzol}]}{100-[\text{Restgehalt an } 4-\text{NO}_2-\text{alkylbenzol}]} \times 100\ (\%)$$

Diese Selektivität gibt den Prozentsatz an, zu dem das umgesetzte 4-Nitro-alkylbenzol in das gewünschte 2-Chlor-4-nitro-alkylbenzol umgewandelt wurde.

Der Chlorverlust wird wie folgt definiert:

$$\text{Chlorverlust} = \frac{[\text{Masse des aus dem Reaktionsgemisch ausgetretenen } Cl_2]}{[\text{Masse des eingeleiteten } Cl_2]} \times 100\ (\%)$$

Der Chlorverlust gibt den Prozentsatz des durch die Lösung geleiteten und unverbraucht gebliebenen und in das Abgas übergehenden Chlors an.

Beispiel 1

(Herstellung von aufchlorierten Co-Katalysatoren)

In 2000 g Perchlorethylen wurden 1,0 Mol des aufzuchlorierenden Co-Katalysators oder Co-Katalysatorgemisches, z.B. 200 g Phenoxathiin suspendiert und mit 3,5 g $FeCl_3$ auf 100°C erhitzt. Unter Rühren leitete man bis zum gewünschten Chloriergrad Chlorgas langsam, z.B. 4,0 Mol ≙ 284 g Chlor in 8 h bis zum Chloriergrad 4,0, ein. Das Perchlorethylen wurde durch Vakuumdestillation entfernt, und der Rückstand wurde umkristallisiert oder umgefällt, z.B. aus Perchlorethylen/Methanol. Nach dem Trocknen bei erhöhter Temperatur, z.B. 100°C im Vakuum, wurde der Chloriergrad durch Elementaranalyse überprüft, z.B. wurden für das so hergestellte Phenoxathiin des Chloriergrades 4,0 folgende Analysenwerte erhalten (Doppelbestimmung):

| | gefunden | berechnet für $C_{12}H_4Cl_4OS$ |
|---|---|---|
| C | 42,7/42,6 % | 42,64 % |
| H | 1,3/ 1,3 % | 1,19 % |
| Cl | 42,2/42,0 % | 41,95 % |
| S | 9,6/ 9,4 % | 9,49 % |

Beispiele 2 - 28

In allen Chlorierbeispielen wurde zum einwandfreien Vergleich der Selektivität und Ausbeute und des Chlorverlustes unter folgenden gleichbleibenden Reaktionsbedingungen gearbeitet: Reaktionstemperatur 90°C ± 2°C; Chlormenge 97-100 Mol-%, bezogen auf p-Alkylnitrobenzol, gasförmig; Zeit der $Cl_2$-Einleitung 5 Stunden bei gleichmäßiger $Cl_2$-Zugabe.

Beispiel 2

(zum Vergleich; Chlorierung mit Eisen-III-chlorid)

Es wurden 100 Gew.-Teile 4-Nitrotoluol in einem Reaktor vorgelegt und mit 0,6 Gew.-Teilen Eisen-III-chlorid versetzt. Man chlorierte unter den vorgenannten Bedingungen. Der Restgehalt an 4-Nitrotoluol betrug 10,3 Gew.-%, die Selektivität für die Bildung von 2-Chlor-4-nitrotoluol betrug 92,7 %. Der Chlorverlust betrug 5,5 % des eingeleiteten Chlors.

Beispiel 3

(zum Vergleich; Chlorierung mit Eisen-III-chlorid und Iod als Co-Katalysator)

Es wurden 100 Gew.-Teile 4-Nitrotoluol in einem Reaktor vorgelegt und mit 0,6 Gew.-Teilen Eisen-III-chlorid und 0,03 Gew.-Teilen des Co-Katalysators elementares Iod versetzt und wie beschrieben chloriert. Der Restgehalt an 4-Nitrotoluol betrug 8,7 Gew.-% die Selektivität für die Bildung von 2-Chlor-4-nitrotoluol betrug 96,8 %. Der Chlorverlust betrug 6,6 % des eingeleiteten Chlors. Im Reaktionsprodukt konnte Iod nachgewiesen werden.

Beispiel 4

Es wurden 100 Gew.-Teile 4-Nitrotoluol in einem Reaktor vorgelegt und mit 0,3 Gew.-Teilen Eisen-III-chlorid und 0,528 Gew.-Teilen des Co-Katalysators der Formel

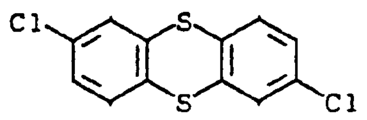

(2,7-Dichlorthianthren)

versetzt und wie beschrieben chloriert. Der Restgehalt an 4-Nitrotoluol betrug 5,9 Gew.-%, die Selektivität für die Bildung von 2-Chlor-4-nitrotoluol betrug 98,3 %. Der Chlorverlust betrug 3,4 % des eingeleiteten Chlors.

Beispiel 5

Es wurden 100 Gew.-Teile 4-Nitrotoluol in einem Reaktor vorgelegt und mit 0,15 Gew.-Teilen Eisen-III-chlorid und 0,339 Gew.-Teilen des Co-Katalysatorgemisches der Formel,

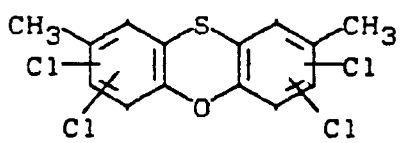

[Tetrachlor-2,8-dimethyl-phenoxathiin (hauptsächlich 1,3,7,9-Tetrachlor-2,8-dimethylphenoxathiin)]

das gemäß Beispiel 1 durch Aufchlorieren von

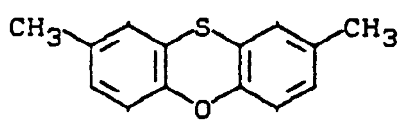

(2,8-Dimethylphenoxathiin)

auf den mittleren Chloriergrad 4,0 hergestellt worden war, versetzt und wie beschrieben chloriert. Der Restgehalt an 4-Nitrotoluol betrug 2,9 Gew.-%, die Selektivität für die Bildung von 2-Chlor-4-nitrotoluol betrug 98,1 %. Der Chlorverlust betrug 0,8 % des eingeleiteten Chlors.

Beispiel 6

Es wurden 100 Gew.-Teile 4-Nitrotoluol in einem Reaktor vorgelegt und mit 0,152 Gew.-Teilen Eisen-III-chlorid und 0,344 Gew.-Teilen des Co-Katalysatorgemisches der Formel,

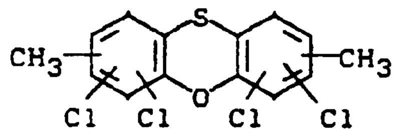

[Tetrachlor-dimethyl-phenoxathiin (hauptsächlich 3,7-Dimethyl-2,4,6,8-tetrachlorphenoxathiin)]

versetzt und wie beschrieben chloriert. Das Co-Katalysatorgemisch war gemäß Beispiel 1 durch Aufchlorieren eines Gemisches aus

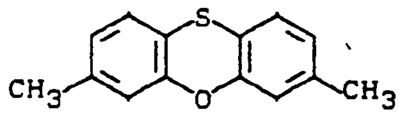

3,7-Dimethylphenoxathiin und

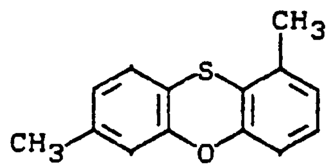

1,7-Dimethylphenoxathiin, das seinerseits aus 3,3'-Dimethyl-diphenylether, Schwefel und $AlCl_3$ hergestellt worden war,

auf den mittleren Chloriergrad 4,0 hergestellt worden. Der Restgehalt an 4-Nitrotoluol betrug 4,3 Gew.-%, die Selektivität für die Bildung von 2-Chlor-4-nitrotoluol betrug 98,4 %. Der Chlorverlust betrug 3,0 % des eingeleiteten Chlors.

Beispiel 7

Es wurden 100 Gew.-Teile 4-Nitrotoluol in einem Reaktor vorgelegt und mit 0,152 Gew.-Teilen Eisen-III-chlorid und 0,318 Gew.-Teilen des Co-Katalysatorgemisches der Formel,

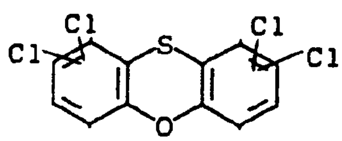

(Tetrachlorphenoxathiin)

das gemäß Beispiel 1 durch Aufchlorieren von

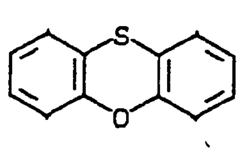

Phenoxathiin

auf den mittleren Chloriergrad 4,0 hergestellt worden war, versetzt und wie beschrieben chloriert. Der Restgehalt an 4-Nitrotoluol betrug 5,3 Gew.-%, die Selektivität für die Bildung von 2-Chlor-4-nitrotoluol betrug 98,7 %. Der Chlorverlust betrug 3,2 % des eingeleiteten Chlors.

Beispiel 8

Es wurden 100 Gew.-Teile 4-Nitrotoluol in einem Reaktor vorgelegt und mit 0,15 Gew.-Teilen Eisen-III-chlorid und 0,446 Gew.-Teilen des Co-Katalysators der Formel

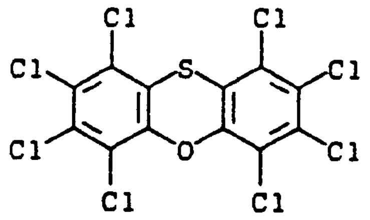

(Octachlorphenoxathiin)

versetzt und wie beschrieben chloriert. Der Restgehalt an 4-Nitrotoluol betrug 3,4 Gew.-%, die Selektivität für die Bildung von 2-Chlor-4-nitrotoluol betrug 98,1 %. Der Chlorverlust betrug 0,7 % des eingeleiteten Chlors.

Beispiel 9

Es wurden 100 Gew.-Teile 4-Nitrotoluol in einem Reaktor vorgelegt und mit 0,301 Gew.-Teilen Eisen-III-chlorid und 0,402 Gew.-Teilen des Co-Katalysators der Formel

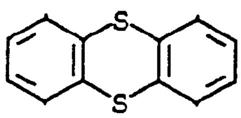

(Thianthren)

versetzt und wie beschrieben chloriert. Der Restgehalt an 4-Nitrotoluol betrug 6,9 Gew.-%, die Selektivität für die Bildung von 2-Chlor-4-nitrotoluol betrug 98,1 %. Der Chlorverlust betrug 3,7 % des eingeleiteten Chlors.

Beispiel 10

Es wurden 100 Gew.-Teile 4-Nitrotoluol in einem Reaktor vorgelegt und mit 0,301 Gew.-Teilen Eisen-III-chlorid und 0,558 Gew.-Teilen des Co-Katalysators der Formel

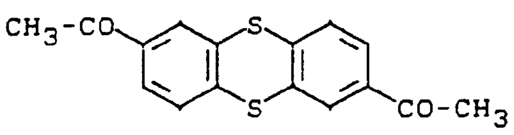

(2,7-Diacetyl-thianthren)

versetzt und wie beschrieben chloriert. Der Restgehalt an 4-Nitrotoluol betrug 9,3 Gew.-%, die Selektivität für die Bildung von 2-Chlor-4-nitrotoluol betrug 98,1 %. Der Chlorverlust betrug 5,2 % des eingeleiteten Chlors.

Beispiel 11

Es wurden 100 Gew.-Teile 4-Nitrotoluol in einem Reaktor vorgelegt und mit 0,30 Gew.-Teilen Eisen-III-chlorid und 0,67 Gew.-Teilen des Co-Katalysators der Formel

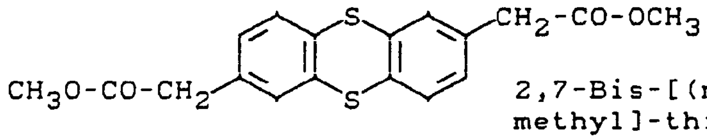

2,7-Bis-[(methoxycarbonyl)-methyl]-thianthren

versetzt und wie beschrieben chloriert. Der Restgehalt an 4-Nitrotoluol betrug 5,9 Gew.-%, die Selektivität für die Bildung von 2-Chlor-4-nitrotoluol betrug 98,1 %. Der Chlorverlust betrug 1,9 %. des eingeleiteten Chlors.

Beispiel 12

Es wurden 100 Gew.-Teile 4-Nitrotoluol in einem Reaktor vorgelegt und mit 0,301 Gew.-Teilen Eisen-III-chlorid und 0,915 Gew.-Teilen des Co-Katalysators der Formel

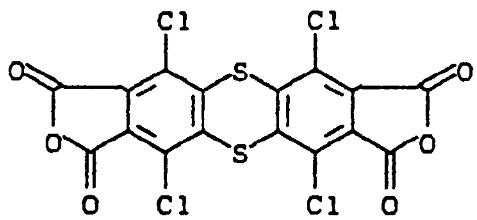

(1,4,6,9-Tetrachlorthianthren-2,3,7,8-tetracarbonsäure-di-anhydrid)

versetzt und wie beschrieben chloriert. Der Restgehalt an 4-Nitrotoluol betrug 4,3 Gew.-%, die Selektivität für die Bildung von 2-Chlor-4-nitrotoluol betrug 96,7 %. Der Chlorverlust betrug 2,0 % des eingeleiteten Chlors.

Beispiel 13

Es wurden 100 Gew.-Teile 4-Nitrotoluol in einem Reaktor vorgelegt und mit 0,15 Gew.-Teilen Eisen-III-chlorid und 0,49 Gew.-Teilen des Co-Katalysators der Formel

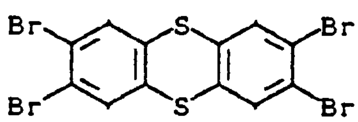

(2,3,7,8-Tetrabromthianthren)

versetzt und wie beschrieben chloriert. Der Restgehalt an 4-Nitrotoluol betrug 6,8 Gew.-%, die Selektivität für die Bildung von 2-Chlor-4-nitrotoluol betrug 97,4 %. Der Chlorverlust betrug 4,1 % des eingeleiteten Chlors.

Beispiel 14

Es wurden 100 Gew.-Teile 4-Nitrotoluol in einem Reaktor vorgelegt und mit 0,301 Gew.-Teilen Eisen-III-chlorid und 0,712 Gew.-Teilen des Co-Katalysatorgemisches der Formel

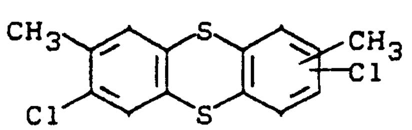

(hauptsächlich 2,7-Dimethyl-3,8-dichlorthianthren und 2,8-Dimethyl-3,7-dichlorthianthren, hergestellt aus ortho-Chlortoluol, Dischwefeldichlorid und $AlCl_3$),

versetzt und wie beschrieben chloriert. Der Restgehalt an 4-Nitrotoluol betrug 6,6 Gew.-%, die Selektivität für

die Bildung von 2-Chlor-4-nitrotoluol betrug 98,1 %. Der Chlorverlust betrug 5,0 % des eingeleiteten Chlors.

Beispiel 15

Es wurden 100 Gew.-Teile 4-Nitrotoluol in einem Reaktor vorgelegt und mit 0,3 Gew.-Teilen Eisen-III-chlorid und 0,429 Gew.-Teilen des Co-Katalysators der Formel

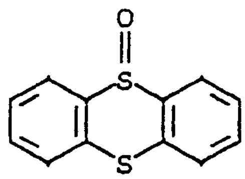

(Thianthren-5-oxid)

versetzt und wie beschrieben chloriert. Der Restgehalt an 4-Nitrotoluol betrug 8,5 Gew.-%, die Selektivität für die Bildung von 2-Chlor-4-nitrotoluol betrug 96,5 %. Der Chlorverlust betrug 6,7 % des eingeleiteten Chlors.

Beispiel 16

Es wurden 100 Gew.-Teile 4-Nitrotoluol in einem Reaktor vorgelegt und mit 0,301 Gew.-Teilen Eisen-III-chlorid und 0,549 Gew.-Teilen des Co-Katalysators der Formel

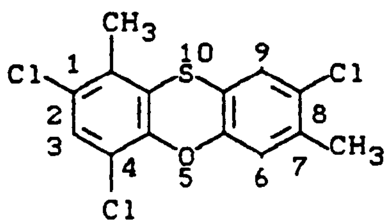

(1,7-Dimethyl-2,4,8-trichlor-phenoxathiin)

versetzt und wie beschrieben chloriert. Der Restgehalt an 4-Nitrotoluol betrug 5,9 Gew.-%, die Selektivität für die Bildung von 2-Chlor-4-Nitrotoluol betrug 98,5 %. Der Chlorverlust betrug 3,8 % des eingeleiteten Chlors.

Beispiel 17

Es wurden 100 Gew.-Teile 4-Nitrotoluol in einem Reaktor vorgelegt und mit 0,15 Gew.-Teilen Eisen-III-chlorid und 0,42 Gew.-Teilen des Co-Katalysatorgemisches der Formel,

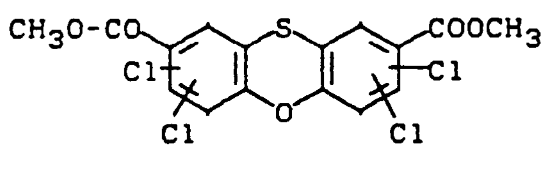

(Tetrachlor-2,8-di(methoxycarbonyl)-phenoxathiin),

das gemäß Beispiel 1 durch Aufchlorieren von

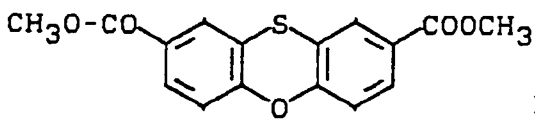

(2,8-Di(methoxycarbonyl)-phenoxathiin)

auf den mittleren Chloriergrad 4,0 hergestellt worden war, versetzt und wie beschrieben chloriert. Der Restgehalt an 4-Nitrotoluol betrug 2,5 Gew.-%, die Selektivität für die Bildung von 2-Chlor-4-nitrotoluol betrug 97,6 %. Der Chlorverlust betrug 0,5 % des eingeleiteten Chlors.

Beispiel 18

Es wurden 100 Gew.-Teile 4-Nitrotoluol in einem Reaktor vorgelegt und mit 0,151 Gew.-Teilen Eisen-III-chlorid und 0,283 Gew.-Teilen des Co-Katalysatorgemisches der Formel,

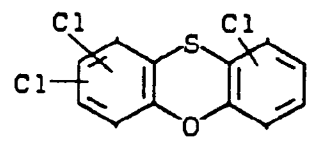

(Trichlorphenoxathiin)

das gemäß Beispiel 1 durch Aufchlorieren von

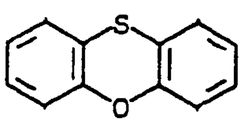

(Phenoxathiin)

auf den mittleren Chloriergrad 3,0 hergestellt worden war, versetzt und wie beschrieben chloriert. Der Restgehalt an 4-Nitrotoluol betrug 6,0 Gew.-%, die Selektivität für die Bildung von 2-Chlor-4-nitrotoluol betrug 98,0 %. Der Chlorverlust betrug 5,3 % des eingeleiteten Chlors.

Beispiel 19

Es wurden 100 Gew.-Teile 4-Nitrotoluol in einem Reaktor vorgelegt und mit 0,3 Gew.-Teilen Eisen-III-chlorid und 0,69 Gew.-Teilen des Co-Katalysatorgemisches der Formel

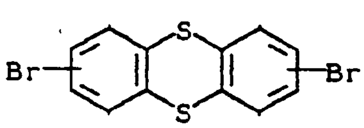

(hauptsächlich 2,7-Dibromthianthren und 2,8-Dibromthianthren),

hergestellt durch Umsetzung von 1 Mol Thianthren mit 2 Mol $Br_2$ in siedendem Eisessig,
versetzt und wie beschrieben chloriert. Der Restgehalt an 4-Nitrotoluol betrug 6,8 Gew.-%, die Selektivität für

die Bildung von 2-Chlor-4-nitrotoluol betrug 97,5 %. Der Chlorverlust betrug 3,4 % des eingeleiteten Chlors.

Beispiel 20

Es wurden 100 Gew.-Teile 4-Nitrotoluol in einem Reaktor vorgelegt und mit 0,3 Gew.-Teilen Eisen-III-chlorid und 0,469 Gew.-Teilen des Co-Katalysators der Formel

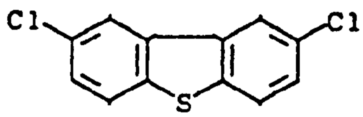

(2,8-Dichlor-dibenzothiophen)

versetzt und wie beschrieben chloriert. Der Restgehalt an 4-Nitrotoluol betrug 7,4 Gew.-%, die Selektivität für die Bildung von 2-Chlor-4-nitrotoluol betrug 98,4 %. Der Chlorverlust betrug 2,9 % des eingeleiteten Chlors.

Beispiel 21

Es wurden 100 Gew.-Teile 4-Nitro-ethylbenzol in einem Reaktor vorgelegt und mit 0,138 Gew.-Teilen Eisen-III-chlorid und 0,311 Gew.-Teilen des Co-Katalysatorgemisches der Formel,

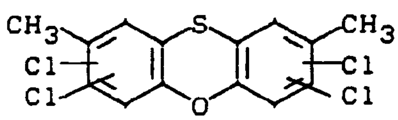

[Tetrachlor-2,8-dimethyl-phenoxathiin, (hauptsächlich 1,3,7,9-Tetrachlor-2,8-dimethylphenoxathiin)],

das gemäß Beispiel 1 durch Aufchlorieren von

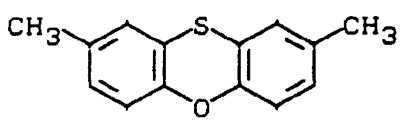

(2,8-Dimethylphenoxathiin)

auf den mittleren Chloriergrad 4,0 hergestellt worden war, versetzt und wie beschrieben chloriert. Der Restgehalt an 4-Nitro-ethylbenzol betrug 8,3 Gew.-%, die Selektivität für die Bildung von 2-Chlor-4-nitrotoluol betrug 97,0 %. Der Chlorverlust betrug 2,6 % des eingeleiteten Chlors.

Beispiel 22

Es wurden 100 Gew.-Teile 4-Nitrotoluol in einem Reaktor vorgelegt und mit 0.300 Gew-Teilen Eisen-III-chlorid und 0.656 Gew.-Teilen des Co-Katalysatorgemisches der Formel

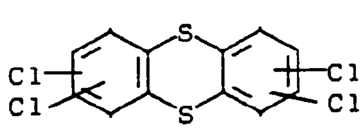

(hauptsächlich Tetrachlorthianthren)

das gemäß Beispiel 1 durch Aufchlorieren von

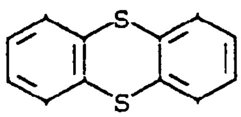

(Thianthren)

auf den mittleren Chloriergrad 4.1 hergestellt worden war, versetzt und wie beschrieben chloriert. Der Restgehalt an 4-Nitrotoluol betrug 5.8 Gew.-%, die Selektivität für die Bildung von 2-Chlor-4-nitrotoluol betrug 98.0%. Der Chlorverlust betrug 2.7% des eingeleiteten Chlors.

Beispiel 23

Es wurden 100 Gew.-Teile 4-Nitrotoluol in einem Reaktor vorgelegt und mit 0.301 Gew.-Teilen Eisen-III-chlorid und 0.513 Gew.-Teilen des Co-Katalysators der Formel

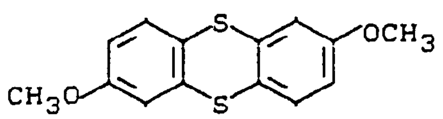

(2.7-Bismethoxy-thianthren)

versetzt und wie beschrieben chloriert. Der Restgehalt an 4-Nitrotoluol betrug 8.4 Gew.-%, die Selektivität für die Bildung von 2-Chlor-4-nitrotoluol betrug 97.0%. Der Chlorverlust betrug 4.5% des eingeleiteten Chlors.

Beispiel 24

Es wurden 100 Gew.-Teile 4-Nitrotoluol in einem Reaktor vorgelegt und mit 0.30 Gew.-Teilen Eisen-III-chlorid und 0.46 Gew.-Teilen des Co-Katalysators der Formel

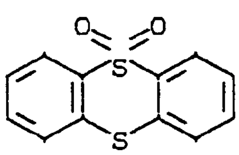

(Thianthren-5,5-dioxid)

versetzt und wie beschrieben chloriert. Der Restgehalt an 4-Nitrotoluol betrug 6.8 Gew.-%, die Selektivität für die Bildung von 2-Chlor-4-nitrotoluol betrug 98.4%. Der Chlorverlust betrug 4.4% des eingeleiteten Chlors.

Beispiel 25

Es wurden 100 Gew.-Teile 4-Nitrotoluol in einem Reaktor vorgelegt und mit 0.300 Gew.-Teilen Eisen-III-chlorid und 0.480 Gew.-Teilen des Co-Katalysators der Formel

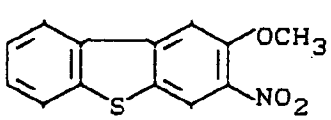

(2-Methoxy-3-nitro-dibenzothiophen)

versetzt und wie beschrieben chloriert. Der Restgehalt an 4-Nitrotoluol betrug 8.3 Gew.-%, die Selektivität für die Bildung von 2-Chlor-4-nitrotoluol betrug 96.6%. Der Chlorverlust betrug 5.2% des eingeleiteten Chlors.

Beispiel 26

Es wurden 100 Gew.-Teile 4-Nitrotoluol in einem Reaktor vorgelegt und mit 0.15 Gew.-Teilen Eisen-III-chlorid und 0.34 Gew.-Teilen des Co-Katalysatorgemisches der Formel

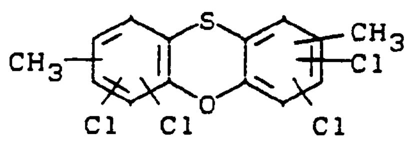

(Dimethyl-tetrachlor-phenoxathiin)

versetzt und wie beschrieben chloriert. Das Co-Katalysatorengemisch war gemäß Beispiel 1 durch Aufchlorieren von einem Gemisch aus

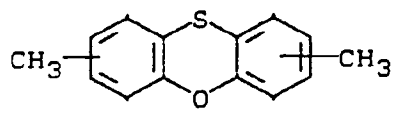

(Dimethylphenoxathiin),

das seinerseits aus einem technischen Gemisch aller möglichen isomerer Ditolylether, Schwefel und $AlCl_3$ hergestellt worden war,)
auf den mittleren Chloriergrad 4.1 hergestellt worden.

Der Restgehalt an 4-Nitrotoluol betrug 4.9 Gew.%, die Selektivität für die Bildung von 2-Chlor-4-nitrotoluol betrug 98.5%. Der Chlorverlust betrug 3.4% des eingeleiteten Chlors.

Beispiel 27

Es wurden 100 Gew.-Teile 4-Nitrotoluol in einem Reaktor vorgelegt und mit 0.300 Gew.-Teilen Eisen-III-chlorid und 0.580 Gew.-Teilen des Co-Katalysators der Formel

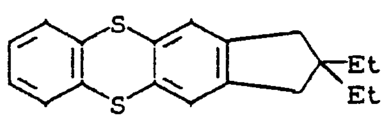

(Diethyl-cyclopentano[6]) -thianthren

versetzt und wie beschrieben chloriert. Der Restgehalt an 4-Nitrotoluol betrug 6.9 Gew.-%, die Selektivität für die Bildung von 2-Chlor-4-nitrotoluol betrug 98.3%. Der Chlorverlust betrug 2.8% des eingeleiteten Chlors.

Beispiel 28

Es wurden 100 Gew.-Teile 4-Nitrotoluol in einem Reaktor vorgelegt und mit 0.301 Gew.-Teilen Eisen-III-chlorid und 0.656 Gew.-Teilen des Co-Katalysatorgemisches der Formel

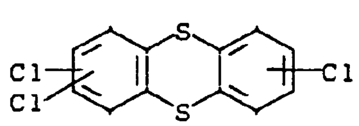

(hauptsächlich Trichlor-thianthren)

das gemäß Beispiel 1 durch Aufchlorieren von

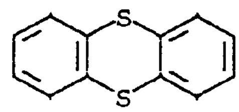

(Thianthren)

auf den mittleren Chloriergrad 3.2 hergestellt worden war, versetzt und wie beschrieben chloriert. Der Restgehalt an 4-Nitrotoluol betrug 7.6 Gew.-%, die Selektivität für die Bildung von 2-Chlor-4-nitro-toluol betrug 98.3%. Der Chlorverlust betrug 4.8% des eingeleiteten Chlors.

## Patentansprüche

1. Verfahren zur Herstellung von 2-Chlor-4-nitroalkylbenzol der Formel

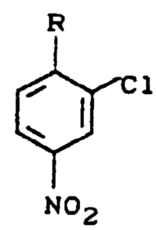

,

in der

R für $C_1$-$C_4$-Alkyl steht,

durch Umsetzung des zugrundeliegenden 4-Nitro-alkylbenzols mit elementarem Chlor oder Chlor abgebenden Verbindungen in Gegenwart eines Friedel-Crafts-Katalysators und eines Co-Katalysators in flüssiger Phase, dadurch gekennzeichnet, daß als Co-Katalysator ein dibenzokondensierter Schwefelheterocyclus der Formel

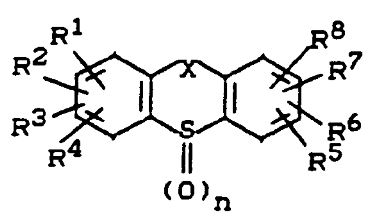

eingesetzt wird, worin

$R^1$, $R^2$, $R^5$ und $R^6$ unabhängig voneinander Wasserstoff, Halogen, Hydroxy, Cyano, Nitro, Amino, Carboxyl, Halogencarbonyl, die Sulfonsäuregruppe, Halogensulfonyl, $R^9$, $OR^9$, $SR^9$, $COR^9$, $OCOR^9$, $COOR^9$, $NHCOR^9$, $SCOR^9$, $SO_2R^9$ oder $SO_2$-$OR^9$ bedeuten, wobei $R^9$ für geradkettiges oder verzweigtes $C_1$-$C_8$-Alkyl, $C_5$-$C_8$-Cycloalkyl, $C_6$-$C_{12}$-Aryl oder $C_7$-$C_{10}$-Aralkyl steht,

$R^3$, $R^4$, $R^7$ und $R^8$ unabhängig voneinander Wasserstoff, Halogen oder geradkettiges oder verzweigtes $C_1$-$C_8$-Alkyl oder durch die Ethergruppe -O- bzw. durch die Estergruppe -OCO- oder -COO- unterbrochenes $C_1$-$C_8$-Alkyl bedeuten,

wobei weiterhin zwei benachbarte Reste von $R^1$ bis $R^4$ bzw. von $R^5$ bis $R^8$ gemeinsam einen anellierten Ring bilden können, der 5 bis 8 Ringglieder aufweisen kann und gesättigt, ungesättigt, aromatisch oder unter Einbeziehung eines oder zweier Atome aus der Gruppe N, O und S heterocyclisch sein kann, und wobei weiterhin zwei benachbarte Reste $R^1$ und $R^2$ bzw. $R^5$ und $R^6$ eine Dicar-

bonsäureanhydridgruppe darstellen können,

X für -O-, -S-, -SO-, $-SO_2-$ oder eine einfache Bindung steht und

n den Wert Null oder Eins annimmt.

2. Verfahren nach Anspruch 1, dadurch gekennzeichnet, daß ein Co-Katalysator der Formel

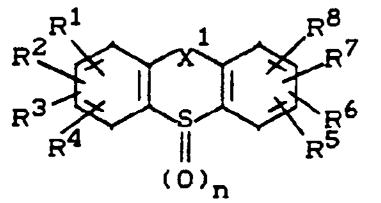

eingesetzt wird, worin

$X^1$ für -O-, -S- oder eine Einfachbindung steht und

$R^1$ bis $R^8$ und n die in Anspruch 1 gegebene Bedeutung haben.

3. Verfahren nach Anspruch 1, dadurch gekennzeichnet, daß ein Co-Katalysator der Formel

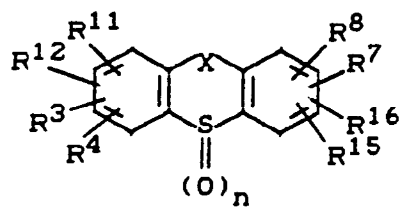

eingesetzt wird, worin

$R^{11}$, $R^{12}$, $R^{15}$ und $R^{16}$ unabhängig voneinander Wasserstoff, Halogen, Cyano, Nitro, Halogencarbonyl, Halogensulfonyl, $R^{19}$, $OR^{19}$, $-COOR^{19}$ oder $COR^{19}$ bedeuten, wobei $R^{19}$ für geradkettiges oder verzweigtes $C_1$-$C_8$-Alkyl, Phenyl oder Benzyl steht, wobei weiterhin zwei benachbare Reste $R^{11}$ und $R^{12}$ bzw. $R^{15}$ und $R^{16}$ eine Dicarbonsäureanhydridgruppe darstellen können, und

$R^3$, $R^4$, $R^7$, $R^8$, X und n die in Anspruch 1 gegebene Bedeutung haben.

4. Verfahren nach Anspruch 3, dadurch gekennzeichnet, daß ein Co-Katalysator der Formel

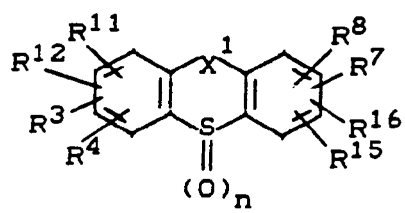

eingesetzt wird, worin

$X^1$ für -O-, -S- oder eine Einfachbindung steht und

$R^{11}$, $R^{12}$, $R^3$, $R^4$, $R^{15}$, $R^{16}$, $R^7$, $R^8$ und n die in Anspruch 3 gegebene Bedeutung haben.

5. Verfahren nach Anspruch 4, dadurch gekennzeichnet, daß ein Co-Katalysator der Formel

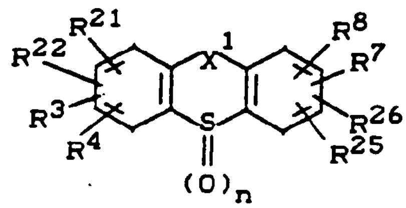

eingesetzt wird, worin

| | |
|---|---|
| $R^{21}$ und $R^{25}$ | unabhängig voneinander Wasserstoff, Fluor, Chlor, Brom, Cyano, Nitro, Fluorcarbonyl, Chlorcarbonyl, Chlorsulfonyl, $R^{29}$ oder $OR^{29}$ bedeuten, wobei $R^{29}$ für geradkettiges oder verzweigtes $C_1$-$C_4$-Alkyl, Phenyl oder Benzyl steht, |
| $R^{22}$ und $R^{26}$ | unabhängig voneinander Wasserstoff, Fluor, Chlor, Brom oder geradkettiges oder verzweigtes $C_1$-$C_4$-Alkyl bedeuten, wobei weiterhin benachbarte Reste $R^{21}$ und $R^{22}$ gemeinsam bzw. $R^{25}$ und $R^{26}$ gemeinsam eine Dicarbonsäureanhydridgruppe darstellen können, und |
| $R^3$, $R^4$, $R^7$, $R^8$, $X^1$ und n | die in Anspruch 4 gegebene Bedeutung haben. |

6. Verfahren nach Anspruch 5, dadurch gekennzeichnet, daß ein Co-Katalysator der Formel

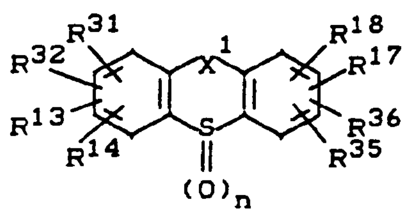

eingesetzt wird, worin

| | |
|---|---|
| $R^{31}$ und $R^{35}$ | unabhängig voneinander Wasserstoff, Fluor, Chlor, Brom, Nitro, Chlorcarbonyl, Methyl, Ethyl, Methoxy, Ethoxy oder Acetyl bedeuten, |
| $R^{32}$ und $R^{36}$ | unabhängig voneinander Wasserstoff, Chlor, Brom, Methyl oder Ethyl bedeuten, wobei weiterhin benachbarte Reste $R^{31}$ und $R^{32}$ gemeinsam bzw. $R^{35}$ und $R^{36}$ gemeinsam eine Dicarbonsäureanhydridgruppe darstellen können, |
| $R^{13}$, $R^{14}$, $R^{17}$ und $R^{18}$ | unabhängig voneinander Wasserstoff, Chlor, Brom, Methyl, $-CH_2-OCH_3$ oder $-CH_2COOCH_3$ bedeuten, |
| $X^1$ | für -O-, -S- oder eine Einfachbindung steht und |
| n | den Wert Null oder Eins annimmt. |

7. Verfahren nach Anspruch 1, dadurch gekennzeichnet, daß 80-110 Mol-%, bevorzugt 80-100 Mol-% Chlor in elementarer Form oder in Form einer Chlor abgebenden Substanz pro Mol 4-Nitro-alkylbenzol eingesetzt werden.

8. Verfahren nach Anspruch 1, dadurch gekennzeichnet, daß Friedel-Crafts-Katalysatoren in einer Menge von 0,005-10 Gew.-%, bevorzugt 0,01-3 Gew.-%, besonders bevorzugt 0,05-1,5 Gew.-%, ganz besonders bevorzugt 0,1-0,75 Gew.-%, bezogen auf die Menge an 4-Nitroalkylbenzol, eingesetzt werden.

9. Verfahren nach Anspruch 1, dadurch gekennzeichnet, daß der Co-Katalysator oder ein Gemisch mehrerer von ihnen in einer Menge von 0,01-10 Gew.-%, bevorzugt 0,01-5 Gew.-%, besonders bevorzugt 0,05-2,5 Gew.-%, ganz besonders bevorzugt 0,1-1 Gew.-%, bezogen auf die Menge an 4-Nitro-alkylbenzol, eingesetzt wird.

10. Verfahren nach Anspruch 1, dadurch gekennzeichnet, daß das molare Verhältnis von Friedel-Crafts-Katalysator zu Co-Katalysator im Bereich von 50:1-1:10, bevorzugt 10:1-1:2, besonders bevorzugt 3:1-1:1,1, eingestellt wird.

## Claims

**1.** Process for the preparation of 2-chloro-4-nitroalkylbenzene of the formula

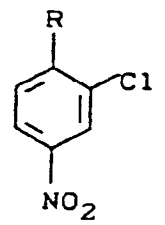

in which

R represents $C_1$-$C_4$-alkyl,

by reaction of the 4-nitro-alkylbenzene on which the product is based with elemental chlorine or chlorine-releasing compounds in the presence of a Friedel-Crafts catalyst and a co-catalyst in the liquid phase, characterized in that the co-catalyst used is a dibenzo-condensed sulphur heterocycle of the formula

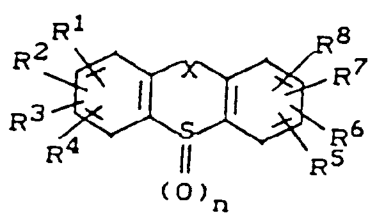

in which

$R^1$, $R^2$, $R^5$ and $R^6$ independently of one another denote hydrogen, halogen, hydroxy, cyano, nitro, amino, carboxyl, halogenocarbonyl, the sulphonic acid group, halogenosulphonyl, $R^9$, $OR^9$, $SR^9$, $COR^9$, $OCOR^9$, $COOR^9$, $NHCOR^9$, $SCOR^9$, $SO_2R^9$ or $SO_2$-$OR^9$, $R^9$ representing straight-chain or branched $C_1$-$C_8$-alkyl, $C_5$-$C_8$-cycloalkyl, $C_6$-$C_{12}$-aryl or $C_7$-$C_{10}$-aralkyl,

$R^3$, $R^4$, $R^7$ and $R^8$ independently of one another denote hydrogen, halogen or straight-chain or branched $C_1$-$C_8$-alkyl, or $C_1$-$C_8$-alkyl interrupted by the ether group -O- or by the ester group -OCO- or -COO,

and additionally two adjacent radicals of $R^1$ to $R^4$ or of $R^5$ to $R^8$ can jointly form a fused ring, which can have 5 to 8 ring members and can be saturated, unsaturated, aromatic or, with the inclusion of one or two atoms from the group N, O and S, heterocyclic, and, furthermore two adjacent radicals $R^1$ and $R^2$ or $R^5$ and $R^6$ can represent a dicarboxylic anhydride group,

X represents -O-, -S-, -SO-, $-SO_2-$ or a single bond and

n assumes the value zero or one.

**2.** Process according to Claim 1, characterized in that a co-catalyst of the formula

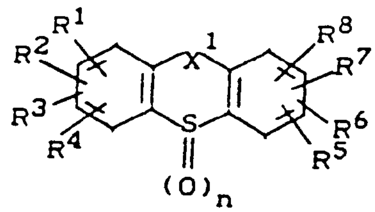

is used, in which

| | |
|---|---|
| $X^1$ | represents -O-, -S- or a single bond and |
| $R^1$ to $R^8$ and n | have the meaning given in Claim 1. |

3. Process according to Claim 1, characterized in that a co-catalyst of the formula

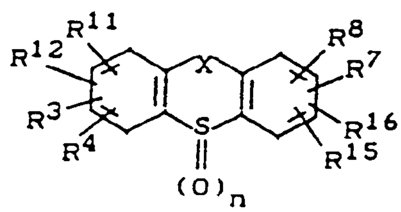

is used, in which

| | |
|---|---|
| $R^{11}$, $R^{12}$, $R^{15}$ and $R^{16}$ | independently of one another denote hydrogen, halogen, cyano, nitro, halogenocarbonyl, halogenosulphonyl, $R^{19}$, $OR^{19}$, $-COOR^{19}$ or $COR^{19}$, $R^{19}$ representing straight-chain or branched $C_1$-$C_8$-alkyl, phenyl or benzyl, and additionally two adjacent radicals $R^{11}$ and $R^{12}$ or $R^{15}$ and $R^{16}$ can represent a dicarboxylic anhydride group, and |
| $R^3$, $R^4$, $R^7$, $R^8$, X and n | have the meaning given in Claim 1. |

4. Process according to Claim 3, characterized in that a co-catalyst of the formula

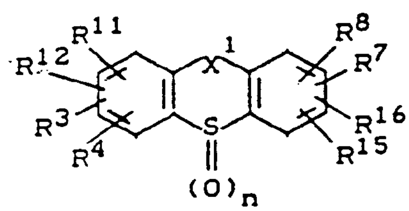

is used, in which

$X_1$ represents -O-, -S- or a single bond and

$R^{11}$, $R^{12}$, $R^3$, $R^4$, $R^{15}$, $R^{16}$, $R^7$, $R^8$ and n have the meaning given in Claim 3.

5. Process according to Claim 4, characterized in that a co-catalyst of the formula

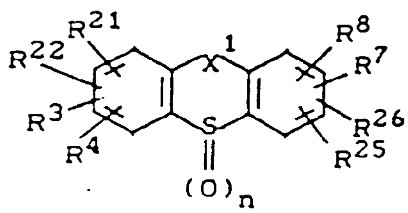

is used, in which

| | |
|---|---|
| $R^{21}$ and $R^{25}$ | independently of one another denote hydrogen, fluorine, chlorine, bromine, cyano, nitro, fluorocarbonyl, chlorocarbonyl, chlorosulphonyl, $R^{29}$ or $OR^{29}$, $R^{29}$ representing straight-chain or branched $C_1$-$C_4$-alkyl, phenyl or benzyl, |
| $R^{22}$ and $R^{26}$ | independently of one another denote hydrogen, fluorine, chlorine, bromine or straight-chain or branched $C_1$-$C_4$-alkyl, and additionally adjacent radicals $R^{21}$ and $R^{22}$ jointly or $R^{25}$ and $R^{26}$ jointly can represent a dicarboxylic anhydride group, and |
| $R^3$, $R^4$, $R^7$, $R^8$, $X^1$ and n | have the meaning given in Claim 4. |

6. Process according to Claim 5, characterized in that a co-catalyst of the formula

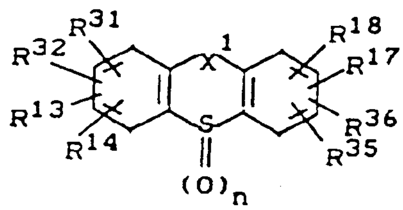

is used, in which

| | |
|---|---|
| $R^{31}$ and $R^{35}$ | independently of one another denote hydrogen, fluorine, chlorine, bromine, nitro, chlorocarbonyl, methyl, ethyl, methoxy, ethoxy or acetyl, |
| $R^{32}$ and $R^{36}$ | independently of one another denote hydrogen, chlorine, bromine, methyl or ethyl, and additionally adjacent radicals $R^{31}$ and $R^{32}$ jointly or $R^{35}$ and $R^{36}$ jointly can represent a dicarboxylic anhydride group, |
| $R^{13}$, $R^{14}$, $R^{17}$ and $R^{18}$ | independently of one another denote hydrogen, chlorine, bromine, methyl, $-CH_2-OCH_3$ or $-CH_2COOCH_3$, |
| $X^1$ | represents -O-, -S- or a single bond and |
| n | assumes the value zero or one. |

7. Process according to Claim 1, characterized in that 80-110 mole %, preferably 80-100 mole % of chlorine in elemental form or in the form of a chlorine-releasing substance are used per mole of 4-nitroalkylbenzene.

8. Process according to Claim 1, characterized in that Friedel-Crafts catalysts are used in an amount of 0.005-10 % by weight, preferably 0.01-3 % by weight, particularly preferentially 0.05-1.5 % by weight and very particularly preferentially 0.1-0.75 % by weight, relative to the amount of 4-nitro-alkylbenzene.

9. Process according to Claim 1, characterized in that the co-catalyst or a mixture of several of these is used in an amount of 0.01-10 % by weight, preferably 0.01-5 % by weight, particularly preferentially 0.05-2.5 % by weight and very particularly preferentially 0.1-1 % by weight, relative to the amount of 4-nitro-alkylbenzene.

10. Process according to Claim 1, characterized in that the molar ratio of Friedel-Crafts catalyst to co-catalyst is adjusted in the range of 50:1-1:10, preferably 10:1-1:2 and particularly preferentially 3:1-1:1.1.

## Revendications

1. Procédé pour la préparation de 2-chloro-4-nitroalkylbenzène répondant à la formule

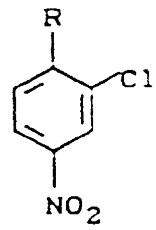

dans laquelle

R représente un groupe alkyle en $C_1$-$C_4$,

par la mise en réaction du 4-nitroalkylbenzène de base avec du chlore élémentaire ou des composés cédant du chlore, en présence d'un catalyseur de Friedel-Craft et d'un cocatalyseur en phase liquide, qui se caractérise par la mise en oeuvre, à titre de cocatalyseur, d'un composé hétérocyclique sulfuré dibenzocondensé répondant à la formule

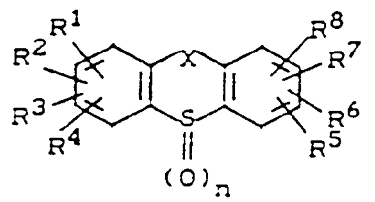

dans laquelle

| | |
|---|---|
| $R^1$, $R^2$, $R^5$ et $R^6$ | représentent, indépendamment l'un de l'autre, un atome d'hydrogène, un atome d'halogène, un groupe hydroxyle, un groupe cyano, un groupe nitro, un groupe amino, un groupe carboxyle, un groupe halogénocarbonyle, le groupe d'acide sulfonique, un groupe halogénosulfonyle, $R^9$, $OR^9$, $SR^9$, $COR^9$, $OCOR^9$, $COOR^9$, $NHCOR^9$, $SCOR^9$, $SO_2R^9$ ou $SO_2OR^9$ où $R^9$ représente un groupe alkyle en $C_1$-$C_8$ à chaîne droite ou ramifiée, un groupe cycloalkyle en $C_5$-$C_8$, un groupe aryle en $C_6$-$C_{12}$ ou un groupe aralkyle en $C_7$-$C_{10}$, |
| $R^3$, $R^4$, $R^7$ et $R^8$ | représentent, indépendamment l'un de l'autre, un atome d'hydrogène, un atome d'halogène ou un groupe alkyle en $C_1$-$C_8$ à chaîne droite ou ramifiée ou encore un groupe alkyle en $C_1$-$C_8$ interrompu par le groupe éther -O- ou par les groupes esters -OCO- ou -COO-, |
| | où, en outre, deux radicaux voisins de $R^1$ à $R^4$ ou de $R^5$ à $R^8$ peuvent former ensemble un noyau condensé qui peut présenter de 5 à 8 termes cycliques et qui peut être saturé, insaturé, aromatique ou encore hétérocyclique en incluant un ou deux atomes du groupe N, O et S, et où, en outre, deux radicaux voisins $R^1$ et $R^2$ ou $R^5$ et $R^6$ peuvent représenter un groupe d'anhydride dicarboxylique, |
| X | représente -O-, -S-, -SO-, $-SO_2$- ou une liaison simple et |
| n | prend la valeur zéro ou un. |

2. Procédé selon la revendication 1, caractérisé en ce qu'on met en oeuvre un cocatalyseur répondant à la formule

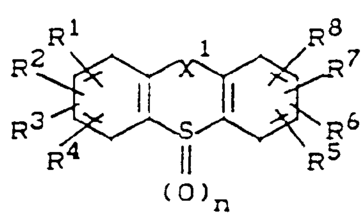

dans laquelle

| | |
|---|---|
| $X^1$ | représente -O-, -S- ou une liaison simple, et |
| $R^1$ à $R^8$ et n | ont la signification indiquée dans la revendication 1. |

3. Procédé selon la revendication 1, caractérisé en ce qu'on met en oeuvre un cocatalyseur répondant à la formule

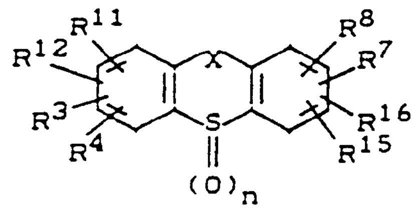

dans laquelle

| | |
|---|---|
| $R^{11}$, $R^{12}$, $R^{15}$ et $R^{16}$ | représentent, indépendamment l'un de l'autre, un atome d'hydrogène, un atome d'halogène, un groupe cyano, un groupe nitro, un groupe halogénocarbonyle, un groupe halogénosulfonyle, $R^{19}$, $OR^{19}$, $COOR^{19}$ ou $COR^{19}$, où $R^{19}$ représente un groupe alkyle en $C_1$-$C_8$ à chaîne droite ou ramifiée, un groupe phényle ou un groupe benzyle et où, en outre, deux radicaux voisins $R^{11}$ et $R^{12}$ ou $R^{15}$ et $R^{16}$ peuvent représenter un groupe d'anhydride dicarboxylique, et |
| $R^3$, $R^4$, $R^7$, $R^8$, X et n | ont la signification indiquée dans la revendication 1. |

4. Procédé selon la revendication 3, caractérisé en ce qu'on met en oeuvre un cocatalyseur répondant à la formule

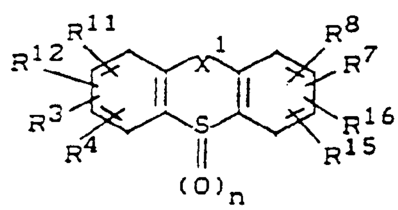

dans laquelle

$X^1$ représente -O-, -S- ou une liaison simple, et

$R^{11}$, $R^{12}$, $R^3$, $R^4$, $R^{15}$, $R^{16}$, $R^7$, $R^8$ et n ont la signification indiquée dans la revendication 3.

5. Procédé selon la revendication 4, caractérisé en ce qu'on met en oeuvre un cocatalyseur de formule

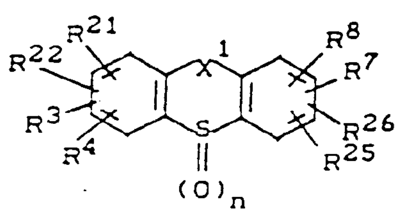

dans laquelle

| | |
|---|---|
| $R^{21}$ et $R^{25}$ | représentent, indépendamment l'un de l'autre, un atome d'hydrogène, un atome de fluor, un atome de chlore, un atome de brome, un groupe cyano, |

| | |
|---|---|
| | un groupe nitro, un groupe fluorocarbonyle, un groupe chlorocarbonyle, un groupe chlorosulfonyle, $R^{29}$ ou $OR^{29}$ où $R^{29}$ représente un groupe alkyle en $C_1$-$C_4$ à chaîne droite ou ramifiée, un groupe phényle ou un groupe benzyle, |
| $R^{22}$ et $R^{26}$ | représentent, indépendamment l'un de l'autre, un atome d'hydrogène, un atome de fluor, un atome de chlore, un atome de brome ou encore un groupe alkyle en $C_1$-$C_4$ à chaîne droite ou ramifiée, où, en outre, des radicaux voisins $R^{21}$ et $R^{22}$ ensemble ou $R^{25}$ et $R^{26}$ ensemble peuvent représenter un groupe d'anhydride dicarboxylique, et |
| $R^3$, $R^4$, $R^7$, $R^8$, $X^1$ et n | ont la signification indiquée dans la revendication 4. |

**6.** Procédé selon la revendication 5, caractérisé en ce qu'on met en oeuvre un cocatalyseur répondant à la formule

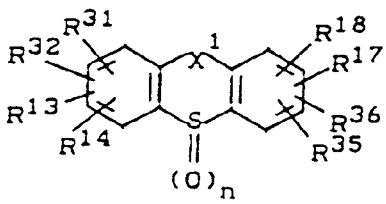

dans laquelle

| | |
|---|---|
| $R^{31}$ et $R^{35}$ | représentent, indépendamment l'un de l'autre, un atome d'hydrogène, un atome de fluor, un atome de chlore, un atome de brome, un groupe nitro, un groupe chlorocarbonyle, un groupe méthyle, un groupe éthyle, un groupe méthoxy, un groupe éthoxy ou un groupe acétyle, |
| $R^{32}$ et $R^{36}$ | représentent, indépendamment l'un de l'autre, un atome d'hydrogène, un atome de chlore, un atome de brome, un groupe méthyle ou un groupe éthyle, où, en outre, des radicaux voisins $R^{31}$ et $R^{32}$ ensemble ou $R^{35}$ et $R^{36}$ ensemble peuvent représenter un groupe d'anhydride dicarboxylique, |
| $R^{13}$, $R^{14}$, $R^{17}$ et $R^{18}$ | représentent, indépendamment l'un de l'autre, un atome d'hydrogène, un atome de chlore, un atome de brome, un groupe méthyle, $-CH_2-OCH_3$ ou $-CH_2COOCH_3$, |
| $X^1$ | représente -O-, -S- ou une liaison simple, et |
| n | prend la valeur zéro ou un. |

**7.** Procédé selon la revendication 1, caractérisé en ce qu'on met en oeuvre de 80 à 110 moles %, de préférence de 80 à 100 moles % de chlore sous forme élémentaire ou sous forme d'une substance cédant du chlore, par mole de 4-nitroalkylbenzène.

**8.** Procédé selon la revendication 1, caractérisé en ce qu'on met en oeuvre des catalyseurs de Friedel-Craft en une quantité de 0,005 à 10% en poids, de préférence de 0,01 à 3% en poids, de manière particulièrement préférée de 0,05 à 1,5% en poids, de manière tout particulièrement préférée de 0,1 à 0,75% en poids, rapportée à la quantité du 4-nitroalkylbenzène.

**9.** Procédé selon la revendication 1, caractérisé en ce qu'on met en oeuvre le cocatalyseur ou un mélange de plusieurs d'entre eux en une quantité de 0,01 à 10% en poids, de préférence de 0,01 à 5% en poids, de manière particulièrement préférée de 0,05 à 2,5% en poids, de manière tout particulièrement préférée de 0,1 à 1% en poids, rapportée à la quantité de 4-nitroalkylbenzène.

**10.** Procédé selon la revendication 1, caractérisé en ce qu'on règle le rapport molaire entre le catalyseur de Friedel-Craft et le cocatalyseur dans le domaine de 50:1-1:10, de préférence de 10:1-1:2, de manière particulièrement préférée de 3:1-1:1,1.